(19) Europäisches Patentamt
European Patent Office
Office européen des brevets

(11) EP 2 762 158 A1

(12) **EUROPEAN PATENT APPLICATION**

(43) Date of publication:
**06.08.2014 Bulletin 2014/32**

(51) Int Cl.:
***A61K 39/00*** *(2006.01)*

(21) Application number: **14000323.7**

(22) Date of filing: **29.01.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.02.2013 JP 2013020799**

(71) Applicant: **NITTO DENKO CORPORATION**
**Osaka 567 (JP)**

(72) Inventors:
- **Okubo, Katsuyuki**
**Ibaraki-shi, Osaka, 567-8680 (JP)**
- **Maeda, Yoshiki**
**Ibaraki-shi, Osaka, 567-8680 (JP)**
- **Shishido, Takuya**
**Ibaraki-shi, Osaka, 567-8680 (JP)**
- **Asari, Daisuke**
**Ibaraki-shi, Osaka, 567-8680 (JP)**
- **Okazaki, Arimichi**
**Ibaraki-shi, Osaka, 567-8680 (JP)**
- **Matsushita, Kyohei**
**Ibaraki-shi, Osaka, 567-8680 (JP)**
- **LI, Wenjing**
**Ibaraki-shi, Osaka, 567-8680 (JP)**
- **Hori, Mitsuhiko**
**Ibaraki-shi, Osaka, 567-8680 (JP)**
- **Sugiyama, Haruo**
**Suita-shi, Osaka, 565-0871 (JP)**

(74) Representative: **Müller-Boré & Partner**
**Patentanwälte PartG mbB**
**Friedenheimer Brücke 21**
**80639 München (DE)**

(54) **E75-vaccine composition for transdermal administration**

(57) The invention provides a cancer vaccine composition for transdermal administration for inducing cellular immunity comprising (i) HER2/neu E75 peptide and/or a modified HER2/neu E75 peptide; and (ii) a first cellular immunity induction promoter.

EP 2 762 158 A1

## Description

### TECHNICAL FIELD

The present invention relates to a cancer vaccine compositions for transdermal administration, comprising HER2 antigen peptides, in particular, HER2/neuE75 peptides and/or modified HER2/neu E75 peptides, and a cellular immunity induction promoter.

### BACKGROUND ART

There are a cancer vaccine that prevents virus infection to prevent a cancer caused by the virus, and a cancer vaccine which provides the result that cancer cells are specifically attacked by the immune system via the recognition of a cancer-specific antigen by the immune mechanism, particularly, the cellular immune mechanism in which cytotoxic T cells (CTL) play an important role. The former is not effective at all for a cancer in which the virus does not participate. The latter is a cancer therapeutic strategy of targeting an antigen possessed by a cancer cell itself. It is considered that the latter is widely effective for cancers having antigen by specifying the antigen. Inter alia, a cancer vaccine based on the viewpoint of the latter can treat tumors that are difficult to remove by surgical operation because of their size, and causes less side effects as compared with the conventional therapies such as chemotherapy and radiation therapy.

HER2/neu gene is amplified and overexpressed in various adenocarcinomas such as breast cancer to overproduce a HER2/neu protein which is an HER2/neu gene product in the cancer cells. The HER2/neu protein is fragmented in the cancer cells to produce HER2/neu peptides which are a partial peptides consisting of 8-12 amino acids. A HER2/neu peptide bound to an MHC class I molecule in the cancer cell is moved to the surface of the cancer cells, and is presented as an antigen bound to the MHC class I molecule on the surface of the cancer cells. Such a presented HER2/neu peptide becomes a mark of cancer cells. Among HER2/neu peptides, HER2/neu E75 peptide which is HLA-A2 or A3 haploid genotype of MHC-restricted peptide has been reported to have effects in clinical study. Therefore, HER2/neu E75 peptide is particularly promising. The amino acid sequence of HER2/neu E75 peptide is Lys-Ile-Phe-Gly-Ser-Leu-Ala-Phe-Leu (SEQ ID NO: 1). When a HER2/neu E75 peptide or a modified HER2/neu E75 peptide in which a part of amino acids of the HER2/neu E75 peptide are substituted or modified is administered to a living body as an antigen (HER2/neu E75 peptide or the modified HER2/neu E75 peptide administered as an antigen is referred as "E75 antigen peptide"), E75 antigen peptide is bound to the MHC Class I molecule on the surface of a dendritic cell which is an antigen presenting cell, or the E75 antigen peptide is once taken into a dendritic cell, bound to the MHC class I molecule in the dendritic cell, and then is moved to the surface of the dendritic cell, and thereby the E75 antigen peptide is presented as an antigen bound to the MHC class I molecule on the surface of the dendritic cell. An activated dendritic cell having E75 antigen peptide/MHC class I molecule complex is moved to the regional lymph node, and activates CD8-positive T lymphocyte which recognizes the E75 antigen peptide/MHC class I molecule complex to differentiate the cells into cytotoxic T cells (CTL). CTL recognizes tumor cells having a complex of HER2/neu E75 peptide (derived from endogenous HER2/neu protein) of the same amino acid sequence as E75 antigen peptide and MHC class I molecule, or tumor cells having a complex of HER2/neu E75 peptide (derived from endogenous HER2/neu protein) of an amino acid sequence having cross-immunoreactivity with E75 antigen peptide and MHC class I molecule, and attacks the recognized tumor cells. Therefore, a HER2/neu E75 peptide and a modified HER2/neu E75 peptide in which a part of amino acids of HER2/neu E75 peptide are substituted or modified are useful as cancer vaccines.

It is also known that an adjuvant for improving the activity of HER2/neu E75 peptide is used as a cancer vaccine. HER2/neu E75 peptide has been studied and reported in relation to immunization by injection. As the adjuvant for the injection, GM-CSF is known.

The most popular administration route of vaccines is subcutaneous or intradermal injection; however, other immunity induction by various administration routes, for example, transdermal administration (Patent Document 1 and Non-Patent Document 1), buccal administration, nasal administration, or sublingual administration (Non-Patent Document 2, Patent Document 2, and Patent Document 3) has been attempted.

### LIST OF DOCUMENTS

[Patent Document 1] US 2008/0193487 A
[Patent Document 2] JP 2002-531415 A
[Patent Document 3] US 2008/0112974 A
[Patent Document 4] JP 07-505883 A
[Patent Document 5] JP 2007-529531 A

[Non-Patent Document 1] Hosoi Akihiro et al., Cancer Research, 68, 3941-3949 (2008)
[Non-Patent Document 2] Zhengrong Cui et al., Pharmaceutical Research, Vol. 19, No. 7, 947-953 (2002)
[Non-Patent Document 3] Mittendorf EA et al., Cancer Immunol Immunother, 57(10), 1511-1521 (2008)
[Non-Patent Document 4] George EP et al., Journal of Clinical Oncology, 23(30), 7536-7545 (2005)

SUMMARY OF THE INVENTION

It is well-known that an adjuvant is used to enhance efficacy of a vaccine. Suitable adjuvants generally vary depending on, for example, the kind of the antigen, the administration route, and the immune response which is desired to be induced (i.e. cellular immunity or humoral immunity). Further, in addition to the adjuvant, there are a variety of substances which promote the induction of the immunity. Therefore, an object of the invention is to provide a cancer vaccine composition having high efficacy and being convenient for use.

A microorganism or a virus itself, or a part thereof is contained in a widely used vaccine and such a vaccine is administered to induce immune response. Generally, since microorganisms or viruses are inhibited from entering a skin because of their sizes, vaccines are required to be administered to a body invasively. Therefore, injections are generally used for immunization. However, injections are not always suitable administration routes because they have problems including pain, fear, injection scar, and subsequent scarring cicatrization. Only health care professionals are premitted to inject them. Intradermal injections having a strong immune effect requires higher techniques for administration. Health care professionals have a risk of a needlestick injury. Patients need to visit the hospital when they are repeatedly administered. Medical wastes such as needles for injection which need to be discarded in a special way generate.

HER2/neu E75 peptide and/or a modified HER2/neu E75 peptide can induce CTL (cytotoxic T cells) via MHC class I molecules. In addition, HER2/neu E75 peptide is a molecule consisting of 9 amino acids having a molecular weight of about 1,000. Although HER2/neu E75 peptide is not a low molecular weight, it is considerably smaller than microorganisms or viruses. Therefore, the possibility for the administration by other administration routes than injection was considered. However, such a formulation has not been developed yet. There are many reasons therefor. For example, suitable cellular immunity induction promoter has been unknown, and it has been unknown whether an antigen can be delivered to tissues suitable for induction of cellular immunity or not. In particular, the cellular immunity induction promoter which can be used when an antigen is administered in order to induce cellular immunity by any administration route other than injection has been unknown.

The inventors searched substances suitable for cellular immunity induction by transdermal administration of HER2/neu E75 peptide and/or a modified HER2/neu E75 peptide, so that the inventors have found that it is preferable to use TLR ligands such as a TLR1/2 ligand, a TLR2 and Dectin1 ligand, a TLR2/6 ligand, a TLR3 ligand, a TLR4 ligand, a TLR5 ligand, a TLR7 and/or a TLR8 ligand, and TLR9 ligand; cyclic dinucleotides such as a cyclic diGMP and a cyclic diAMP; immunomodulatory small molecule drugs such as bestatin, pidotimod, and levamisole hydrochloride; cyclooxygenase inhibitors such as etodolac and loxoprofen; prostaglandin receptor antagonists such as an EP2 receptor antagonist, an EP4 receptor antagonist, a DP receptor antagonist, and an IP receptor antagonist; prostaglandin receptor agonist such as an EP3 receptor agonist; TSLP production inhibitor such as berberine chloride and naringenin; adenylate cyclase inhibitors such as 2',5'-dideoxyadenosine; omega-3 fatty acids such as eicosapentaenoic acid and docosahexaenoic acid; PPAR agonists such as a PPAR-α agonist, a PPAR-5 agonist, and a PPAR-γ agonist; dopamine receptor antagonists such as a D1 receptor antagonist, and a D5 receptor antagonist; dopamine receptor agonists such as a D2 receptor agonist, a D3 receptor agonist, a D4 receptor agonist; histamine receptor antagonists such as an H1 receptor antagonist, and an H2 receptor antagonist; histamine receptor agonists such as an H1 receptor agonist, an H3 receptor agonist, and an H4 receptor agonist; serotonin receptor antagonists such as a 5-HT2 receptor antagonist, a 5-HT4 receptor antagonist, a 5-HT6 receptor antagonist, and a 5-HT7 receptor antagonist; serotonin receptor agonists such as a 5-HT1 receptor agonist, and a 5-HT2 receptor agonist; vasopressin receptor antagonists such as a V2 receptor antagonist; vasopressin receptor agonists such as a V1 receptor agonist; muscarine receptor antagonists such as an M1 receptor antagonist, an M3 receptor antagonist, and an M5 receptor antagonist; muscarine receptor agonists such as an M1 receptor agonist, an M2 receptor agonist, an M3 receptor agonist, an M4 receptor agonist, and an M5 receptor agonist; adrenergic receptor antagonists such as an α1 receptor antagonist, a β1 receptor antagonist, a β2 receptor antagonist, and a β3 receptor antagonist; adrenergic receptor agonists such as an α1 receptor agonist, and an α2 receptor agonist; angiotensin receptor agonists such as an AT2 receptor agonist; GABA receptor agonists such as a $GABA_B$ receptor agonist; thrombin receptor antagonists such as a PAR-1 receptor antagonist; thrombin receptor agonist such as a PAR-1 receptor agonist; opioid receptor agonists such as buprenorphine; leukotriene receptor antagonist such as a CysLT1 receptor antagonist, and a CysLT2 receptor antagonist; leukotriene receptor agonist such as a BLT receptor agonist; ADP receptor agonists such as adenosine diphosphate; melatonin receptor agonist such as melatonin; somatostatin receptor agonist such as octreotide; cannabinoid receptor agonist such as dronabinol; sphingosine-1 phosphate receptor agonists such as fingolimod; metabotropic glutamate receptor agonists such as an mGluR2 receptor agonist, an mGluR3 receptor agonist, an mGluR4 receptor agonist, an mGluR6 receptor agonist, an mGluR7 receptor

agonist, and an mGluR8 receptor agonist; phospholipase A2 inhibitors such as glycyrrhizic acid; TGF-β production inhibitors such as pirfenidone; Th2 cytokine inhibitors such as suplatast tosilate; and pharmacologically acceptable acids such as decanoic acid, lauric acid, myristic acid, isostearic acid, and oleic acid, and pharmacologically acceptable salts thereof. Further, the inventors have found that cellular immunity induction by transdermal administration of HER2/neu E75 peptide and/or the modified HER2/neu E75 peptide can be promoted by using a helper peptide such as Peptide-25 instead of or in addition to the above mentioned substances. That is, the inventors have found that cellular immunity can be remarkably improved by a combination of a TLR ligand and a helper peptide, a combination of a cyclic dinucleotide and a helper peptide, a combination of an immunomodulatory small molecule drug and a helper peptide, a combination of a cyclooxygenase inhibitor and a helper peptide, a combination of a prostaglandin receptor antagonist and a helper peptide, a combination of a prostaglandin receptor agonist and a helper peptide, a combination of a TSLP production inhibitor and a helper peptide, a combination of an adenylate cyclase inhibitor and a helper peptide, a combination of an omega-3 fatty acid and a helper peptide, a combination of a PPAR agonist and a helper peptide, a combination of a dopamine receptor antagonist and a helper peptide, a combination of a dopamine receptor agonist and a helper peptide, a combination of a histamine receptor agonist and a helper peptide, a combination of a histamine receptor antagonist and a helper peptide, a combination of a serotonin receptor agonist and a helper peptide, a combination of a serotonin receptor antagonist and a helper peptide, a combination of a vasopressin receptor antagonist and a helper peptide, a combination of a vasopressin receptor agonist and a helper peptide, a combination of a muscarine receptor antagonist and a helper peptide, a combination of a muscarine receptor agonist and a helper peptide, a combination of an adrenergic receptor antagonist and a helper peptide, a combination of an adrenergic receptor agonist and a helper peptide, a combination of an angiotensin receptor agonist and a helper peptide, a combination of a GABA receptor agonist and a helper peptide, a combination of a thrombin receptor antagonist and a helper peptide, a combination of a thrombin receptor agonist and a helper peptide, a combination of an opioid receptor agonist and a helper peptide, a combination of an ADP receptor agonist and a helper peptide, a combination of a leukotriene receptor antagonist and a helper peptide, a combination of a leukotriene receptor agonist and a helper peptide, a combination of a melatonin receptor agonist and a helper peptide, a combination of a somatostatin receptor agonist and a helper peptide, a combination of a cannabinoid receptor agonist and a helper peptide, a combination of a sphingosine-1 phosphate receptor agonist and a helper peptide, a combination of a metabotropic glutamate receptor agonist and a helper peptide, a combination of a phospholipase A2 inhibitor and a helper peptide, a combination of a TGF-β production inhibitor and a helper peptide, a combination of a Th2 cytokine inhibitor and a helper peptide, or a combination of a pharmacologically acceptable acid or a pharmacologically acceptable salt thereof and a helper peptide.

Further, the inventors have found that higher cellular immunity induction effect can be obtained by administering the composition under mildly irritating condition. Specifically, high cellular immunity induction effect is obtained by selecting a mildly irritating condition under which the transepidermal water loss (TEWL) ($g/h\cdot m^2$) of model animals for evaluating skin irritation has 50 or less before administering the cancer vaccine composition for transdermal administration, and then administering the cancer vaccine composition for transdermal administration to the animal. Alternatively, high cellular immunity induction effect can be obtained by selecting a mildly irritating condition under which cutaneous TSLP level (pg/mg protein) is 10, 000 or less as evaluated by model animals for evaluating skin irritation after administering the cancer vaccine composition for transdermal administration.

Therefore, in a first aspect, the invention provides aspects as listed below:

(1) A cancer vaccine composition for transdermal administration for inducing cellular immunity, comprising:

(i) HER2/neu E75 peptide and/or a modified HER2/neu E75 peptide; and

(ii) a first cellular immunity induction promoter selected from a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, and a combination of two or more of them;

(2) The cancer vaccine composition for transdermal administration according to (1), further comprising a pharmacologically acceptable acid or a pharmacologically acceptable salt thereof as a second cellular immunity induction

promoter;

(3) The cancer vaccine composition for transdermal administration according to (1) or (2), wherein the first cellular immunity induction promoter is a TLR ligand;

(4) The cancer vaccine composition for transdermal administration according to (1) or (2), wherein the first cellular immunity induction promoter is a cyclic dinucleotide;

(5) The cancer vaccine composition for transdermal administration according to (1) or (2), wherein the first cellular immunity induction promoter is an immunomodulatory small molecule drug;

(6) The cancer vaccine composition for transdermal administration according to (1) or (2), wherein the first cellular immunity induction promoter is a cyclooxygenase inhibitor;

(7) The cancer vaccine composition for transdermal administration according to (1) or (2), wherein the first cellular immunity induction promoter is a prostaglandin receptor antagonist, and wherein the prostaglandin receptor antagonist is an EP2 receptor antagonist, an EP4 receptor antagonist, a DP receptor antagonist, or an IP receptor antagonist;

(8) The cancer vaccine composition for transdermal administration according to (1) or (2), wherein the first cellular immunity induction promoter is a prostaglandin receptor agonist, and wherein the prostaglandin receptor agonist is an EP3 receptor agonist;

(9) The cancer vaccine composition for transdermal administration according to (1) or (2), wherein the first cellular immunity induction promoter is a TSLP production inhibitor;

(10) The cancer vaccine composition for transdermal administration according to (1) or (2), wherein the first cellular immunity induction promoter is an adenylate cyclase inhibitor;

(11) The cancer vaccine composition for transdermal administration according to (1) or (2), wherein the first cellular immunity induction promoter is an omega-3 fatty acid;

(12) The cancer vaccine composition for transdermal administration according to (1) or (2), wherein the first cellular immunity induction promoter is a PPAR agonist, and the PPAR agonist is a PPAR-$\alpha$ agonist, a PPAR-$\delta$ agonist, or a PPAR-$\gamma$ agonist;

(13) The cancer vaccine composition for transdermal administration according to (1) or (2), wherein the first cellular immunity induction promoter is a dopamine receptor antagonist, and wherein the dopamine receptor antagonist is a D1 receptor antagonist, or a D5 receptor antagonist;

(14) The cancer vaccine composition for transdermal administration according to (1) or (2), wherein the first cellular immunity induction promoter is a dopamine receptor agonist, and wherein the a dopamine receptor agonist is a D2 receptor agonist, a D3 receptor agonist, or a D4 receptor agonist;

(15) The cancer vaccine composition for transdermal administration according to (1) or (2), wherein the first cellular immunity induction promoter is a histamine receptor antagonist, and wherein the histamine receptor antagonist is an H1 receptor antagonist, or an H2 receptor antagonist;

(16) The cancer vaccine composition for transdermal administration according to (1) or (2), wherein the first cellular immunity induction promoter is a histamine receptor agonist, and wherein the histamine receptor agonist is an H1 receptor agonist, an H3 receptor agonist, or an H4 receptor agonist;

(17) The cancer vaccine composition for transdermal administration according to (1) or (2), wherein the first cellular immunity induction promoter is a serotonin receptor antagonist, and wherein the serotonin receptor antagonist is a 5-HT2 receptor antagonist, a 5-HT4 receptor antagonist, a 5-HT6 receptor antagonist, or a 5-HT7 receptor antagonist;

(18) The cancer vaccine composition for transdermal administration according to (1) or (2), wherein the first cellular immunity induction promoter is a serotonin receptor agonist, and wherein the serotonin receptor agonist is a 5-HT1 receptor agonist or a 5-HT2 receptor agonist;

(19) The cancer vaccine composition for transdermal administration according to (1) or (2), wherein the first cellular immunity induction promoter is a vasopressin receptor antagonist, and wherein the vasopressin receptor antagonist is a V2 receptor antagonist;

(20) The cancer vaccine composition for transdermal administration according to (1) or (2), wherein the first cellular immunity induction promoter is a vasopressin receptor agonist, and wherein the vasopressin receptor agonist is a V1 receptor agonist;

(21) The cancer vaccine composition for transdermal administration according to (1) or (2), wherein the first cellular immunity induction promoter is a muscarine receptor antagonist, and wherein the muscarine receptor antagonist is an M1 receptor antagonist, an M3 receptor antagonist, or an M5 receptor antagonist;

(22) The cancer vaccine composition for transdermal administration according to (1) or (2), wherein the first cellular immunity induction promoter is a muscarine receptor agonist, and wherein the muscarine receptor agonist is an M1 receptor agonist, an M2 receptor agonist, an M3 receptor agonist, an M4 receptor agonist, or an M5 receptor agonist;

(23) The cancer vaccine composition for transdermal administration according to (1) or (2), wherein the first cellular immunity induction promoter is an adrenergic receptor antagonist, and wherein the adrenergic receptor antagonist is an $\alpha$1 receptor antagonist, a $\beta$1 receptor antagonist, a $\beta$2 receptor antagonist, or a $\beta$3 receptor antagonist;

(24) The cancer vaccine composition for transdermal administration according to (1) or (2), wherein the first cellular immunity induction promoter is an adrenergic receptor agonist, and wherein the adrenergic receptor agonist is an α1 receptor agonist, or an α2 receptor agonist;
(25) The cancer vaccine composition for transdermal administration according to (1) or (2), wherein the first cellular immunity induction promoter is an angiotensin receptor agonist, and wherein the angiotensin receptor agonist is an AT2 receptor agonist;
(26) The vaccine composition for transdermal administration according to (1) or (2), wherein the first cellular immunity induction promoter is a GABA receptor agonist, and wherein the GABA receptor agonist is a $GABA_B$ receptor agonist;
(27) The cancer vaccine composition for transdermal administration according to (1) or (2), wherein the first cellular immunity induction promoter is a thrombin receptor antagonist, and wherein the thrombin receptor antagonist is a PAR-1 receptor antagonist;
(28) The cancer vaccine composition for transdermal administration according to (1) or (2), wherein the first cellular immunity induction promoter is a thrombin receptor agonist, and wherein the thrombin receptor agonist is a PAR-1 receptor agonist;
(29) The cancer vaccine composition for transdermal administration according to (1) or (2), wherein the first cellular immunity induction promoter is an opioid receptor agonist;
(30) The cancer vaccine composition for transdermal administration according to (1) or (2), wherein the first cellular immunity induction promoter is a leukotriene receptor antagonist, and wherein the leukotriene receptor antagonist is a CysLT1 receptor antagonist, or a CysLT2 receptor antagonist;
(31) The cancer vaccine composition for transdermal administration according to (1) or (2), wherein the first cellular immunity induction promoter is a leukotriene receptor agonist, and wherein the leukotriene receptor agonist is a BLT receptor agonist.
(32) The cancer vaccine composition for transdermal administration according to (1) or (2), wherein the first cellular immunity induction promoter is a melatonin receptor agonist.
(33) The cancer vaccine composition for transdermal administration according to (1) or (2), wherein the first cellular immunity induction promoter is a somatostatin receptor agonist.
(34) The cancer vaccine composition for transdermal administration according to (1) or (2), wherein the first cellular immunity induction promoter is a cannabinoid receptor agonist.
(35) The cancer vaccine composition for transdermal administration according to (1) or (2), wherein the first cellular immunity induction promoter is a sphingosine-1 phosphate receptor agonist.
(36) The cancer vaccine composition for transdermal administration according to (1) or (2), wherein the first cellular immunity induction promoter is a metabotropic glutamate receptor agonist, and wherein the metabotropic glutamate receptor agonist is an mGluR2 receptor agonist, an mGluR3 receptor agonist, an mGluR4 receptor agonist, an mGluR6 receptor agonist, an mGluR7 receptor agonist, or an mGluR8 receptor agonist.
(37) The cancer vaccine composition for transdermal administration according to (1) or (2), wherein the first cellular immunity induction promoter is an ADP receptor agonist;
(38) The cancer vaccine composition for transdermal administration according to (1) or (2), wherein the first cellular immunity induction promoter is a phospholipase A2 inhibitor;
(39) The cancer vaccine composition for transdermal administration according to (1) or (2), wherein the first cellular immunity induction promoter is a TGF-β production inhibitor;
(40) The cancer vaccine composition for transdermal administration according to (1) or (2), wherein the first cellular immunity induction promoter is a Th2 cytokine inhibitor;
(41) The cancer vaccine composition for transdermal administration according to (1) or (2), wherein the first cellular immunity induction promoter is a helper peptide;
(42) The cancer vaccine composition for transdermal administration according to (1) or (2), wherein the first cellular immunity induction promoter is a combination of one or more substances selected from the group consisting of a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor and a Th2 cytokine inhibitor, and a helper peptide;
(43) The cancer vaccine composition for transdermal administration according to any one of (1)-(42), wherein the

composition is administered under mildly irritating condition;

(44) The cancer vaccine composition for transdermal administration according to (43), wherein the mildly irritating condition is a condition under which transepidermal water loss (TEWL) of a model animal for evaluating skin irritation before administration is 50 g/h.$m^2$ or less;

(45) The cancer vaccine composition for transdermal administration according to (43) or (44), wherein the mildly irritating condition is a condition under which TSLP level in the skin of a model animal for evaluating skin irritation after administration is 10,000 pg/mg protein or less; and

(46) A cancer vaccine tape preparation for transdermal administration, comprising: a support; and an adhesive layer disposed on one side of the support, wherein the adhesive layer comprises the cancer vaccine composition according to any one of (1)-(45);

(47) A cancer vaccine liquid formulation for external use for transdermal administration, comprising the cancer vaccine composition according to any one of (1)-(45), wherein the formulation is in a form of liquid formulation for external use;

(48) A cancer vaccine cream formulation for transdermal administration, comprising the cancer vaccine composition according to any one of (1)-(45), the formulation being a form of cream formulation.

In another aspect, the cancer vaccine of the invention can be used for treating or preventing a cancer. Therefore, the invention also provides aspects as listed below:

(49) A method for treating or preventing a cancer, comprising administrating a therapeutically effective amount of (i) HER2/neu E75 peptide and/or a modified HER2/neu E75 peptide, and (ii) a cellular immunity induction promoter selected from a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, and a combination of two or more of them to a subject transdermally; and

(50) A method for treating or preventing a cancer, comprising administrating a therapeutically effective amount of a cancer vaccine composition for transdermal administration according to any one of (1)-(45), a cancer vaccine tape preparation for transdermal administration according to (46), or a cancer vaccine liquid formulation for external use for transdermal administration according to (47), or a cancer vaccine cream formulation for transdermal administration according to (48) to a subject.

In another aspect, the invention also provides a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, a combination of two or more of them, as the cellular immunity induction promoter of the HER2/neu E75 peptide and/or the modified HER2/neu E75 peptide. Therefore, the invention also provides the following aspect.

(51) A TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine

receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-$\beta$ production inhibitor, a Th2 cytokine inhibitor, or a combination of two or more them, for using as the cellular immunity induction promoter of the HER2/neu E75 peptide and/or the modified HER2/neu E75 peptide for transdermal administration.

The present invention also provides the following embodiments:

(52) A method of inducing cellular immunity, which comprises transdermally administering to a subject (i) HER2/neu E75 peptide and/or a modified HER2/neu E75 peptide, and (ii) a first cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more of them;

(53) TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor or a combination of two or more of them, for use in promoting the induction of cellular immunity by transdermal administration of HER2/neu E75 peptide and/or a modified HER2/neu E75 peptide;

(54) A combination of (i) HER2/neu E75 peptide and/or a modified HER2/neu E75 peptide and (ii) a first cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more of them, for use in inducing cellular immunity by transdermal administration of HER2/neu E75 peptide and/or a modified HER2/neu E75 peptide;

(55) A combination of (i) HER2/neu E75 peptide and/or a modified HER2/neu E75 peptide and (ii) a cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more of them for use in the treatment or prevention of a cancer, the composition is transdermally administered; and

(56) Use of (i) HER2/neu E75 peptide and/or a modified HER2/neu E75 peptide and (ii) a cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more of them, for the manufacture of a cancer vaccine composition for transdermal administration.

Since the cancer vaccine composition of the present invention can be transdermally administered, it has the following advantages: excellent compliance, for example, non-invasive administration, no pain, and release from fear of injection; patients can administer the cancer vaccine composition by himself/herself since the administration is simple; a risk of accidental infection due to needlestick injury by health care workers can be avoided; in the case of repetitive administration, the ambulatory frequency can be reduced, and this can contribute to the improvement in quality of life of the patient; and medical wastes which necessitate special disposition such as an injection needle are not generated. In addition, if the cancer vaccine composition is in a form of a patch preparation such as a cataplasm preparation or a tape preparation, a predetermined dose can be surely administered, the drug releasing rate can be arbitrarily controlled, and the cancer vaccine composition is not adhered to other site upon administration. Further, since the patch preparation can be easily detached, in the case where a side effect is generated, the patient himself/herself can instantaneously stop the administration by removing the patch from the application site. Further, there is also an advantage that efficacy of the cancer vaccine composition of the present invention is remarkably improved, as compared with administration of the HER2/neu E75 peptide and/or the modified HER2/neu E75 peptide alone. Further, the cancer vaccine composition of the present invention also has an advantage that transdermal administration of the composition induces stronger cellular immunity as compared with injection administration.

First, terms used in the present specification will be defined so that the present invention can be more easily understood. Terms having no definition have the meaning which is normally understood by a person skilled in the art in the fields of, particularly, medicine, pharmacy, immunology, cell biology, biochemistry, polymer chemistry and the like, unless the context requires otherwise.

I. Definition

As used herein, the term "HER2/neu E75 peptide" means a peptide derived from a product of a cancer gene HER2/neu (a HER2 protein), which consists of a sequence Lys Ile Phe Gly Ser Leu Ala Phe Leu (SEQ ID NO: 1).

As used herein, term "modified HER2/neu E75 peptide" means a peptide in which all or a part of amino acids of the HER2/neu E75 peptide are modified by substitution or modification and the like. The modified HER2/neu E75 peptide includes, for example,

(a) a peptide consisting of an amino acid sequence in which one to several, for example, one, two, three, four or five amino acids are substituted, deleted or added in the amino acid sequence of the HER2/neu E75 peptide; and

(b) a peptide consisting of an amino acid sequence in which all or a part of amino acids, for example, one or more, for example, one, two, three, four, five, six, seven, eight, nine, ten, eleven or twelve amino acids are modified in the amino acid sequence of the HER2/neu E75 peptide.

Examples of "modification" of an amino acid which can be possessed by a modified HER2/neu E75 peptide include, but not limited to, aliphatic chain addition modification such as alkylation such as acetylation and methylation, glycosylation, hydroxylation, carboxylation, aldehydization, phosphorylation, sulfonylation, formylation, myristoylation, palmitoylation and stearoylation, octanoylation, esterification, amidation, deamidation, disulfide bond formation modification such as cystine modification, glutathione modification and thioglycolic acid modification, glycation, ubiquitination, succinimide formation, glutamylation, prenylation and the like. The modified HER2/neu E75 peptide may contain a

combination of substitution, deletion or addition of one or more amino acids, and modification of one or more amino acids.

The HER2/neu E75 peptide and/or the modified HER2/neu E75 peptide peptide can be in the free form, or any pharmacologically acceptable salt form, for example, a form of acid salts (acetic acid salt, TFA salt, hydrochloric acid salt, sulfuric acid salt, phosphoric acid salt, lactic acid salt, tartaric acid salt, maleic acid salt, fumaric acid salt, oxalic acid salt, hydrobromic acid salt, succinic acid salt, nitric acid salt, malic acid salt, citric acid salt, oleic acid salt, palmitic acid salt, propionic acid salt, formic acid salt, benzoic acid salt, picric acid salt, benzenesulfonic acid salt, dodecylsulfuric acid salt, methanesulfonic acid salt, p-toluenesulfonic acid salt, glutaric acid salt, a variety of amino acid salts etc.), metal salts (alkali metal salts (e.g. sodium salt, potassium salt), alkaline earth metal salts (e.g. calcium salt, magnesium salt), aluminum salt etc.), or amine salts (triethylamine salt, benzylamine salt, diethanolamine salt, t-butylamine salt, dicyclohexylamine salt, arginine salt, dimethylammonium salt, ammonium salt etc.). A preferable pharmacologically acceptable salt is an acetic acid salt or a TFA salt. The HER2/neu E75 peptide and/or the modified HER2/neu E75 peptide which has been synthesized or produced, isolated and purified by a well-known method can be used.

As used herein, the term "cellular immunity induction promoter" means any substance which can enhance the cellular immune response induced by an antigen which is administered together with the substance, as compared with the immune response induced by the antigen without the substance. The cellular immunity induction promoter may include substances specified in the present specification, though it is not limited by the action mechanism by which induction of the cellular immunity is promoted.

As used herein, the term "TLR ligand" means a ligand for a Toll-like receptor (TLR), for example, including a ligand for TLR1-9. The TLR ligand includes a ligand for a heterodimer of TLR1 and TLR2 (a TLR1/2 ligand), a ligand for a heterodimer of TLR2 and TLR6 (a TLR2/6 ligand), a ligand for TLR2 and Dectin1, a TLR3 ligand, a TLR4 ligand, a TLR5 ligand, a TLR7 ligand and/or a TLR8 ligand, and a TLR9 ligand, all of which can be used as the cellular immunity induction promoter of the invention. In a preferred aspect of the present invention, the TLR ligand is a TLR1/2 ligand, a TLR2 and Dectin1 ligand, a TLR3 ligand, a TLR4 ligand, a TLR7 ligand and/or a TLR8 ligand, and/or a TLR9 ligand.

As used herein, the term "TLR1/2 ligand" means a ligand of a heterodimer of a Toll-like receptor (TLR) 1 and a Toll-like receptor (TLR) 2, and includes, for example, a triacylated lipoprotein derived from a bacterial cell wall and a salt thereof, and these may be an extract, a product or a synthetic product, and are not limited to them.

In a preferable aspect of the present invention, the TLR1/2 ligand is $Pam_3CSK_4$. $Pam_3CSK_4$ has the formula:I

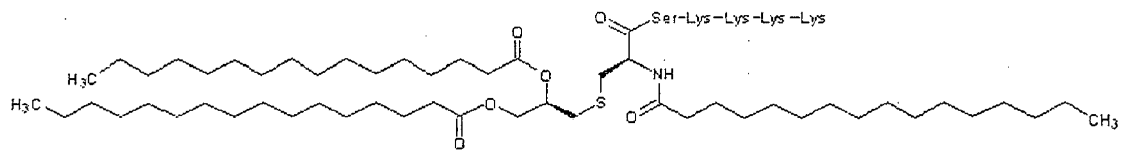

As used herein, the term "TLR2 and Dectin1 ligand" means a ligand of a Toll-like receptor (TLR) 2 and a β1, 3-glucan receptor (Dectin1), and includes, for example, a β1,3-glucan derived from a fungal cell wall and a salt thereof, and these may be an extract, a product or a synthetic product, and are not limited to them. In a preferable aspect of the present invention, the TLR2 and Dectin1 ligand is Zymosan derived from a yeast cell wall.

As used herein, the term "TLR3 ligand" means a ligand of a Toll-like receptor (TLR) 3, and includes, for example, a double-stranded RNA (dsRNA) derived from a virus and a salt thereof, and these may be an extract, a product or a synthetic product, and are not limited to them. In a preferable aspect of the present invention, the TLR3 ligand is polyinosinic-polycytidylic acid (Poly(I:C)) which is a synthetic product and/or a salt thereof.

As used herein, the term "TLR4 ligand" means a ligand of a Toll-like receptor (TLR) 4, and includes, for example, a lipopolysaccharide (LPS) derived from a bacterium or a plant, particularly, a lipid A derivative, for example, monophosphoryl lipid A, a 3 deacylated monophosphoryl lipid A (3D-MPL), OM174, OM 294 DP or OM 197 MP-Ac DP and the like, alkyl glucosaminide phosphate (AGP), for example, AGP disclosed in WO 98/50399 or US 6303347, or a salt of AGP as disclosed in US 6764840, and a lipopolysaccharide derived from a Pantoea bacterium, a glucopyranosyl lipid, and sodium hyaluronate, but is not limited to them.

In a preferable aspect of the present invention, as the TLR4 ligand, lipopolysaccharides derived from genus Acetobacter (e.g. Acetobacter aceti, Acetobacter xylinum, Acetobacter orientalis etc.), genus Zymomonas (e.g. Zymomonas mobilis etc.), genus Xanthomonas (e.g. Xanthomonas campestris etc.), genus Enterobacter (e.g. Enterobacter cloacae etc.), and genus Pantoea (e.g. Pantoea agglomerans etc.) are preferable. Extracts derived from these lipopolysaccharides or purified lipopolysaccharides can be used as they are. In addition, for example, lipopolysaccharides (IP-PAl) derived from Pantoea agglomerans can be purchased from Funakoshi Corporation. In addition, in a preferable aspect of the present invention, the TLR4 ligand is a lipopolysaccharide derived from a Pantoea bacterium, glucopyranosyl lipid, and/or sodium hyaluronate.

As used herein, the term "TLR7 and/or TLR8 ligand" means a ligand for a Toll-like receptor (TLR) 7 and/or TLR8, for example, including a single-stranded RNA, imiquimod, resiquimod (R848), TLR7-II, and other compounds,

for example, loxoribine and bropirimine, without limitation.

As used herein, the term "TLR7 and/or TLR8 ligand" means a ligand of a Toll-like receptor (TLR) 7 and/or TLR8, and includes, for example, a single-stranded RNA, imiquimod, resiquimod (R848), TLR7-II and other compounds, for example, loxoribine and bropirimine, but is not limited to them.

In a preferred aspect of the invention, the TLR7 ligand and/or the TLR8 ligand is imiquimod. Imiquimod is 1-(2-methylpropyl)-1H-imidazo[4,5-c]quinoline-4-amine having the formula:

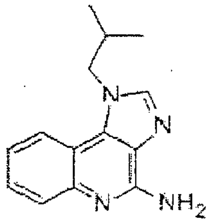

For example, the characteristics and a production process thereof are described in JP 7-505883 A (Patent Document 4).

In another preferred aspect, the TLR7 ligand and/or the TLR8 ligand is resiquimod. Resiquimod is 4-amino-2-(ethoxymethyl)-α,α-dimethyl-1H-imidazo[4,5-c]quinoline-1-et hanol having the formula:

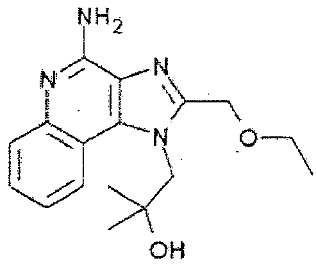

In another preferred aspect, the TLR7 ligand and/or the TLR8 ligand is TLR7-II. TLR7-II is represented by the formula:

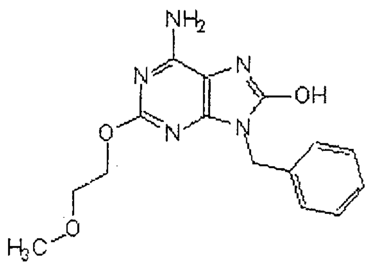

In another preferred aspect, the TLR7 ligand and/or the TLR8 ligand is bropirimine. Bropirimine is represented by the formula:

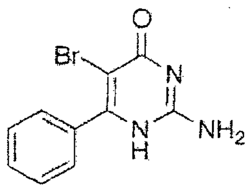

As used herein, the term "TLR9 ligand" means a ligand of a Toll-like receptor (TLR) 9, and includes, for example, ODN1826 and the like. The TLR9 ligand used in the present invention may be an extract, a product or a synthetic product, and is not limited to them. In a preferable aspect of the present invention, the TLR9 ligand is ODN1826.

ODN1826 is an oligodeoxynucleotide consisting of the following sequence (SEQ ID NO: 2):

5'-tccatgacgttcctgacgtt-3'

As used herein, the term "TLR2/6 ligand" means a ligand of a heterodimer of Toll-like receptor (TLR) 2 and a Toll-like receptor (TLR) 6, and includes, for example, a diacylated lipoprotein derived from a cell wall of mycoplasma and a salt thereof, and these may be an extract, a product or a synthetic product, and are not limited to them. In a preferable aspect of the present invention, the TLR2/6 ligand is $Pam_2CSK_4$, MALP-2 and/or FSL-1.

$Pam_2$ $CSK_4$ is represented by the following formula:

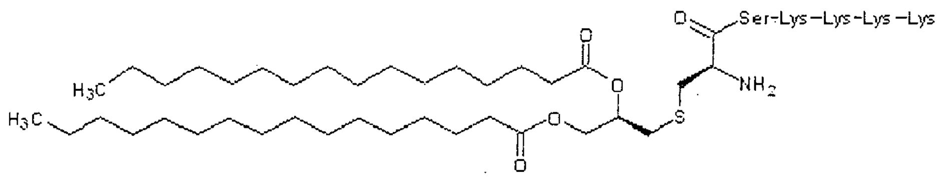

FSL-1 is represented by the following formula:

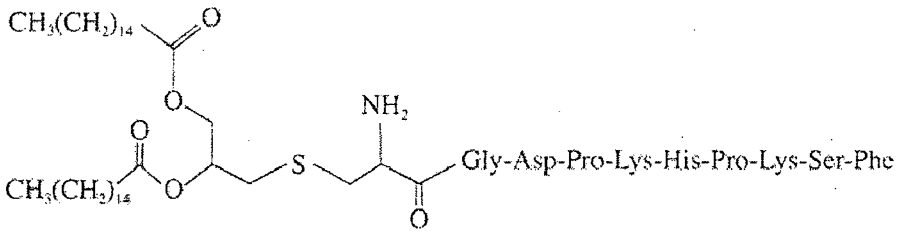

As used herein, the term "TLR5 ligand" means a ligand of a Toll-like receptor (TLR) 5, and includes, for example, flagellin and the like. The TLR5 ligand used in the present invention may be an extract, a product or a synthetic product, and is not limited to them. In a preferable aspect of the present invention, the TLR5 ligand is flagellin.

The Toll-like receptor (TLR) is a family of type I transmembrane proteins which initiates congenital immune response in which a specific cytokine, a specific chemokine and a growth factor participate, by in vivo activation thereof. All TLRs can activate a certain intracellular signal transmission molecule, for example, a nuclearity factor κB (NF-κB) and a mitogen-activated protein kinase (MAP kinase) or the like, while a specific population of a cytokine and a chemokine which are released seems to be inherent to each TLR. TLR3, 7, 8 and 9 include a subfamily of TLR which is present in an endosome fraction or a lysosome fraction of an immune cell (e.g. dendritic cell and monocyte). Specifically, TLR3 is expressed by a wide range of cells such as a dendritic cell and a fibroblast, TLR7 is expressed by a plasma cell-like dendritic cell, and is expressed by a monocyte to a lesser extent, TLR8 is expressed by a monocyte as well as a monocyte-derived dendritic cell and a myeloid dendritic cell, and TLR9 is expressed by a plasma cell-like dendritic cell. This subfamily mediates recognition of a microorganism nucleic acid (single-stranded RNA, double-stranded RNA, single-stranded DNA etc.). Agonists of TLR3, TLR7 and/or TLR8, and TLR9 stimulate production of a variety of inflammatory cytokines (including, for example, interleukin-6, interleukin-12, TNF-α, and interferon-γ). Such agonists also promote increase in expression of a costimulatory molecule (e.g. CD40, CD80, and CD86), a major histocompatibility complex molecule, and a chemokine receptor. Type I interferons (IFNα and IFNβ) are produced by a cell upon activation with TLR7 and/or TLR8 agonists.

As used herein, the term "cyclic dinucleotide" means a molecule in which two OH groups of a sugar part of two nucleotides produce an ester for each same phosphoric acid molecule, and thereby nucleotides are cyclized, and an analog thereof, and includes, for example, cyclic di-AMP(c-di-AMP), cyclic di-GMP (c-di-GMP), c-dGpGp, c-dGpdGp, c-GpAp, c-GpCp, c-GpUp and the like, but is not limited to them. The cyclic dinucleotide activates a dendritic cell or a T cell. Further examples of the cyclic dinucleotide, use of them as an adjuvant, and a process for producing them are described in JP 2007-529531 A (Patent Document 5). In a preferable aspect of the present invention, the cyclic dinucleotide is cyclic di-GMP and/or cyclic di-AMP. The cyclic di-GMP has the formula:

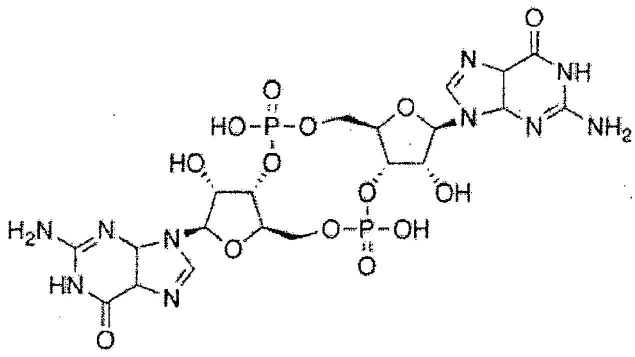

and the process for its synthesis is described in Kawai et al., Nucleic Acids Research Suppl.3: 103-4.

As used herein, the term "helper peptide" means any peptide which activates helper T cells, including, for example, a helper peptide derived from tuberculosis, a helper peptide derived from measles virus, a helper peptide derived from Hepatitis B virus, a helper peptide derived from hepatitis C virus, a helper peptide derived from Chlamydia trachomatis, a helper peptide derived from P. falciparum sporozoite, a helper peptide derived from keyhole limpet haemocyanin, a helper peptide derived from tetanus toxin, a helper peptide derived from pertussis toxin, a helper peptide derived from diphtheria toxin, a helper peptide derived from cancer cell (for example, an IMA-MMP-001 helper peptide, a CEA-006 helper peptide, an MMP-001 helper peptide, a TGFBI-004 helper peptide, an HER-2/neu (aa776-790) helper peptide, an AE36 helper peptide, an AE37 helper peptide, an MET-005 helper peptide, a BIR-002 helper peptide), and universal helper analogs (for example, PADRE). In a preferable aspect of the present invention, the helper peptide consists of 10 to 18 amino acids, preferably 12 to 16 amino acids, more preferably 13 to 15 amino acids. In a preferred aspect of the present invention, the helper peptide is Peptide-25 or PADRE. Peptide-25 is a peptide of 15 amino acids consisting of a sequence Phe Gln Asp Ala Tyr Asn Ala Gly His Asn Ala Val Phe (SEQ ID NO: 3), the sequence corresponds to the amino acid residue 240-254 of Ag85B, the major protein secreted by human-type tuberculosis (Mycobacterium tuberculosis). PADRE is a peptide of 13 amino acids consisting of a sequence D-Ala Lys cyclohexyl-Ala Val Ala Ala Trp Thr Leu Lys Ala Ala D-Ala (Herein referred as SEQ ID NO: 4).

Further, in the invention, instead of the helper peptide, or in combination of the helper peptide, any peptide modified by substitution or modification of all or some amino acids of the helper peptide (hereinafter referred as "modified helper peptide") can also be used.

The modified helper peptide includes, for example,

(a) a peptide consisting of an amino acid sequence in which one to several, for example, one, two, three, four or five amino acids are substituted, deleted or added, in an amino acid sequence of the original helper peptide; and
(b) a peptide consisting of an amino acid sequence in which all or a part of amino acids, for example, one or more, for example, one, two, three, four, five, six, seven, eight, nine, ten, eleven, twelve, thirteen, fourteen, or fifteen amino acids are modified, in an amino acid sequence of the original helper peptide.

Examples of the "modification" of an amino acid which can be possessed by the modified helper peptide include, but are not limited to, aliphatic chain addition modification such as acetylation, alkylation such as methylation, glycosylation, hydroxylation, carboxylation, aldehydization, phosphorylation, sulfonylation, formylation, addition of fatty acid such as myristoylation, palmitoylation and stearoylation, octanoylation, esterification, amidation, deamidation, disulfide bond formation modification such as cystine modification, glutathione modification and thioglycolic acid modification, glycation, ubiquitination, succinimide formation glutamylation, prenylation and the like. In addition, the modified helper peptide may contain a combination of substitution, deletion or addition of one or more amino acids, and modification of one or more amino acids.

As used herein, the term "cyclooxygenase inhibitor" means a substance which inhibits the function of cyclooxygenase (COX). This is also referred to as "COX inhibitor" hereinafter. As COX inhibitors, there are a COX inhibitor which selectively acts on particular cyclooxygenase (e.g. COX-1 or COX-2), and a COX inhibitor having no selectivity. Examples of COX inhibitors which can be used in the present invention include etodolac, loxoprofen, celecoxib, valdecoxib, parecoxib, lumiracoxib, meloxicam, tenoxicam, diclofenac, mefenamic acid, tolfenamic acid, flufenamic acid, meclofenamic acid, niflumic acid, benzydamine, indobufen, triflusal, tolmetin, fenoprofen, tiaprofenic acid, felbinac, nepafenac, amfenac, pravadoline, zaltoprofen, sulindac, nabumetone, diflunisal, piroxicam, ibuprofen, naproxen, fenoprofen, aspirin, methyl salicylate, salicylamide, salsalate, aloxiprin, tolmetin, indomethacin, proglumetacine, acemetacin, flurbiprofen, pranoprofen, acetaminophen, floctafenine, lornoxicam, tenoxicam, tiaprofenic acid, oxaprozin, ketoprofen, dexketoprofen, dexibuprofen, alminoprofen, ketorolac, mofezolac, phenylbutazone, oxyphenylbutazone, ketophenylbutazone,

feprazone, phenbutazone, ethenzamide, tiaramide, tinoridine, epirizole, emorfazone and a derivative thereof, as well as a pharmacologically acceptable salt thereof. In a preferable aspect of the present invention, the COX inhibitor is etodolac and/or loxoprofen.

Loxoprofen is represented by the formula:

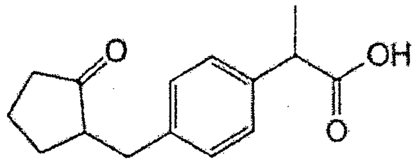

As used herein, the term "prostaglandin receptor antagonist" means a substance having the function of preventing prostaglandin from acting on a receptor, and includes, for example, an EP2 receptor antagonist, an EP4 receptor antagonist, a DP receptor antagonist, and an IP receptor antagonist.

As used herein, the term "EP2 receptor antagonist" means a substance having the function of preventing prostaglandin E2 from acting on an EP2 receptor. Examples of the EP2 receptor antagonist include AH6809 and a derivative thereof, as well as a pharmacologically acceptable salt thereof.

AH6809 is represented by the formula:

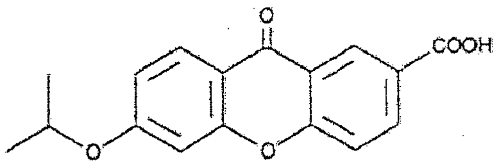

As used herein, the term "EP4 receptor antagonist" means a substance having the function of preventing prostaglandin $E_2$ from acting on an EP4 receptor. Examples of the EP4 receptor antagonist include GW627368X and a derivative thereof, as well as a pharmacologically acceptable salt thereof.

GW627368X is represented by the formula:

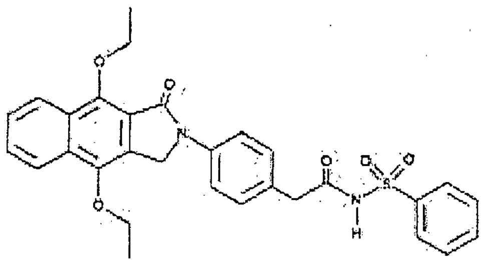

As used herein, the term "DP receptor antagonist" means a substance having the function of preventing prostaglandin $D_2$ from acting on a DP receptor. Examples of the DP receptor antagonist include S-5751, BWA868C and a derivative thereof, as well as a pharmacologically acceptable salt thereof.

BWA868C is represented by the formula:

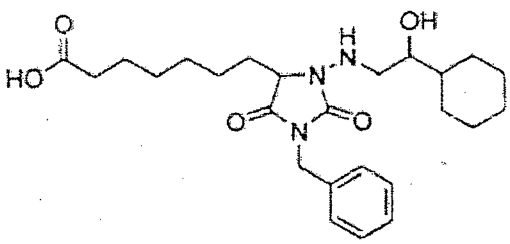

As used herein, the term "IP receptor antagonist" means a substance having the function of preventing prostaglandin $I_2$ from acting on an IP receptor. Examples of the IP receptor antagonist include R01138452 and a derivative

thereof, as well as a pharmacologically acceptable salt thereof.

R01138452 is represented by the formula:

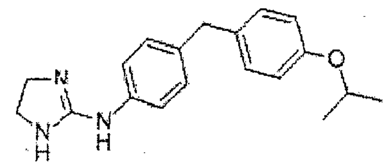

As used herein, the term "prostaglandin receptor agonist" means a substance having the function of acting on a prostaglandin receptor, and includes, for example, an EP3 receptor agonist.

As used herein, the term "EP3 receptor agonist" means a substance having the function of acting on an EP3 receptor. Examples of the EP3 receptor agonist include sulprostone, GR63799, cloprostenol, ONO-AE-248, carbacyclin, and a derivative thereof, as well as a pharmacologically acceptable salt thereof.

Sulprostone is represented by the formula:

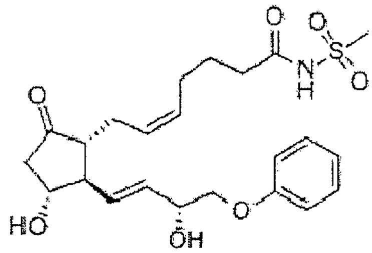

As used herein, the term "TSLP production inhibitor" means a substance having the function of inhibiting production of TSLP. Since a drug which inhibits NF-κB is thought to indirectly inhibit the production of TSLP, it is included in this category. Examples of the TSLP production inhibitor include naringenin, berberine, resveratrol, luteolin, apigenin, chrysoeriol, velutin, rutin, hesperidin, quercetin, daidzein, genistein, noscapine, diindolylmethane, xanthone, parthenolide and a derivative thereof, as well as a pharmacologically acceptable salt thereof.

Berberine is represented by the formula:

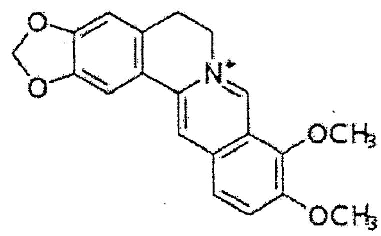

As used herein, the term "adenylate cyclase inhibitor" means a substance having the function of inhibiting the activity of adenylate cyclase. Examples of the adenylate cyclase inhibitor include 2',5'-dideoxyadenosine, niacin, insulin, and a derivative thereof, as well as a pharmacologically acceptable salt thereof.

2',5'-dideoxyadenosine is represented by the formula:

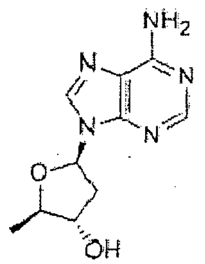

As used herein, the term "omega-3 fatty acid" refers to an unsaturated fatty acid having a carbon-carbon double bond at the ω-3 position. Examples of the omega-3 fatty acid include eicosapentaenoic acid, α-linolenic acid, docosahexaenoic acid, and a derivative thereof, as well as a pharmacologically acceptable salt thereof.

Eicosapentaenoic acid is represented by the formula:

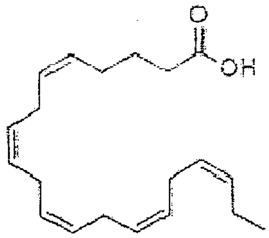

As used herein, the term "PPAR agonist" means a substance having the function of acting on a peroxisome proliferator-activated receptor, and includes, for example, a PPAR-α agonist, a PPAR-δ agonist, and a PPAR-γ agonist.

As used herein, the term "PPAR-α agonist" means a substance having the function of acting on an α type peroxisome proliferator-activated receptor. The term "PPAR-δ agonist" means a substance having the function of acting on a δ type peroxisome proliferator-activated receptor. The term "PPAR-γ agonist" means a substance having the function of acting on a γ type peroxisome proliferator-activated receptor. Examples of the PPAR-α agonist, and/or the PPAR-δ agonist, and/or the PPAR-γ agonist include clofibrate, fenofibrate, bezafibrate, ciprofibrate, etofibrate, telmisartan, oleyl ethanolamide, tetradecylthioacetic acid, troglitazone, pioglitazone, rosiglitazone, balaglitazone, rivoglitazone, ciglitazone, darglitazone, edaglitazone, netoglitazone, indeglitazar, tesaglitazar, muraglitazar, aleglitazar, and a derivative thereof, as well as a pharmacologically acceptable salt thereof.

Clofibrate is represented by the formula:

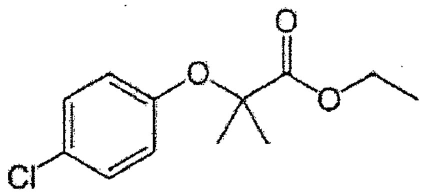

As used herein, the term "dopamine receptor antagonist" means a substance having the function of preventing dopamine from acting on a receptor, and includes, for example, a D1 receptor antagonist, and a D5 receptor antagonist.

As used herein, the term "D1 receptor antagonist" means a substance having the function of preventing dopamine from acting on a D1 receptor. Examples of the D1 receptor antagonist include benzazepine, fenoldopam, lorcaserin, SCH23390, SCH39166, LE300 and a derivative thereof, as well as a pharmacologically acceptable salt thereof.

Benzazepine is represented by the formula:

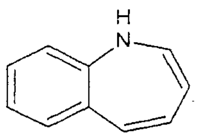

As used herein, the term "D5 receptor antagonist" means a substance having the function of preventing dopamine from acting on a D5 receptor. Examples of the D5 receptor antagonist include SCH39166 and a derivative thereof, as well as a pharmacologically acceptable salt thereof.

SCH39166 is represented by the formula:

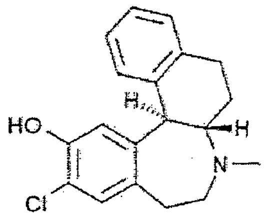

As used herein, the term "dopamine receptor agonist" means a substance having the function of acting on a dopamine receptor, and includes, for example, a D2 receptor agonist, a D3 receptor agonist, and a D4 receptor agonist.

As used herein, the term "D2 receptor agonist" means a substance having the function of acting on a D2 receptor. Examples of the D2 receptor agonist include cabergoline, bromocriptine, pergolide, ropinirole, talipexole, aripiprazole, lurasidone, and a derivative thereof, as well as a pharmacologically acceptable salt thereof.

Ropinirole is represented by the formula:

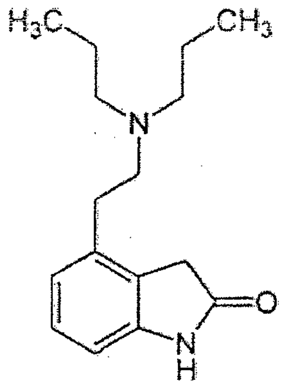

As used herein, the term "D3 receptor agonist" means a substance having the function of acting on a D3 receptor. Examples of the D3 receptor agonist include piribedil, rotigotine, PD1289077, OH-DPAT and a derivative thereof, as well as a pharmacologically acceptable salt thereof.

Rotigotine is represented by the formula:

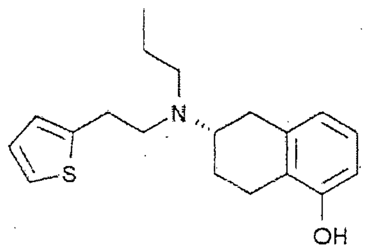

As used herein, the term "D4 receptor agonist" means a substance having the function of acting on a D4 receptor. Examples of the D4 receptor agonist include flibanserin, ABT724, PD168077, CP226269 and a derivative thereof, as well as a pharmacologically acceptable salt thereof.

Flibanserin is represented by the formula:

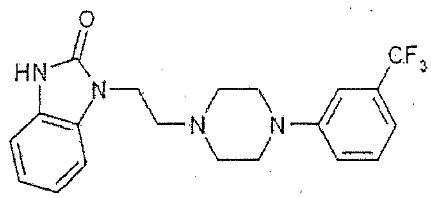

As used herein, the term "histamine receptor antagonist" means a substance having the function of preventing histamine from acting on a receptor, and includes, for example, a H1 receptor antagonist, and a H2 receptor antagonist.

As used herein, the term "H1 receptor antagonist" means a substance having the function of preventing histamine from acting on a H1 receptor. Examples of the H1 receptor antagonist include ketanserin, thonzylamine, mepyramine, tripelenamine, dimethindene, clemastine, bamipine, isothipendyl, chlorphenoxamine, dimetotiazine, chlorpromazine, hydroxyzine, opipramol, betahistine, cinnarizine, levocabastine, antazoline, diphenylpyraline, carbinoxamine, doxylamine, alimemazine, cyclizine, meclozine, levocetirizine, cyproheptadine, phenindamine, triprolidine, azatadine, astemizole, terfenadine, acrivastine, ebastine, desloratadine, rupatadine, bilastine, mizolastine, noberastine, rocastine, temelastine, bepotastine, diphenhydramine, chlorpheniramine, ketotifen, promethazine, cyproheptadine, epinastine, olopatadine, bepotastine, astemizole, emedastine, mequitazine, oxatomide, loratadine, fexofenadine, cetirizine, azelastine, and a derivative thereof, as well as a pharmacologically acceptable salt thereof.

Diphenhydramine is represented by the formula:

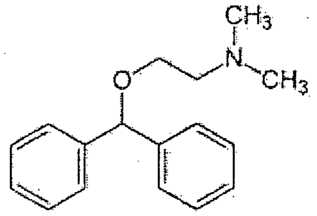

As used herein, the term "H2 receptor antagonist" means a substance having the function of preventing histamine from acting on a H2 receptor. Examples of the H2 receptor antagonist include cimetidine, ranitidine, famotidine, nizatidine, roxatidine, lafutidine, and a derivative thereof, as well as a pharmacologically acceptable salt thereof.

Famotidine is represented by the formula:

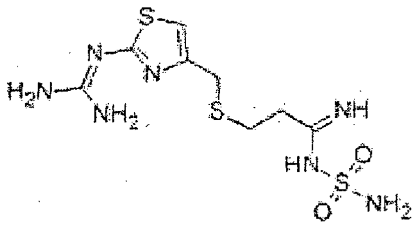

As used herein, the term "histamine receptor agonist" means a substance having the function of acting on a histamine receptor, and includes, for example, a H1 receptor agonist, a H3 receptor agonist, and a H4 receptor agonist.

As used herein, the term "H1 receptor agonist" means a substance having the function of acting on a H1 receptor. Examples of the H1 receptor agonist include 2-pyridylethylamine, 2-thiazolylethylamine and a derivative thereof, as well as a pharmacologically acceptable salt thereof.

2-pyridylethylamine is represented by the formula:

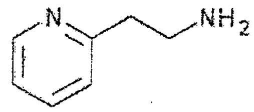

As used herein, the term "H3 receptor agonist" means a substance having the function of acting on a H3 receptor. Examples of the H3 receptor agonist include Immethridine, Imetit, Immepip, $\alpha$-methylhistamine, proxyfan, and a derivative thereof, as well as a pharmacologically acceptable salt thereof.

Proxifan is represented by the formula:

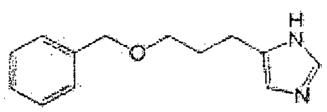

As used herein, the term "H4 receptor agonist" means a substance having the function of acting on a H4 receptor. Examples of the H4 receptor agonist include 4-methylhistamine, VUF8430, immepip and a derivative thereof, as well as a pharmacologically acceptable salt thereof.

4-methylhistamine is represented by the formula:

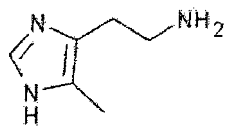

As used herein, the term "serotonin receptor antagonist" means a substance having the function of preventing serotonin from acting on a receptor, and includes, for example, a 5-HT2 receptor antagonist, a 5-HT4 receptor antagonist, a 5-HT6 receptor antagonist, and a 5-HT7 receptor antagonist.

As used herein, the term "5-HT2 receptor antagonist" means a substance having the function of preventing serotonin from acting on a 5-HT2 receptor. Examples of the 5-HT2 receptor antagonist include pizotifen, risperidone, olanzapine, quetiapine, aripiprazole, blonanserin, clozapine, paliperidone, ritanserin, yohimbine, mesulergine, agomelatine, cyclobenzaprine, sarpogrelate, methysergide, ketanserin, and a derivative thereof, as well as a pharmacologically acceptable salt thereof.

Olanzapine is represented by the formula:

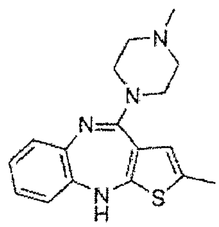

As used herein, the term "5-HT4 receptor antagonist" means a substance having the function of preventing serotonin from acting on a 5-HT4 receptor. Examples of the 5-HT4 receptor antagonist include piboserod, GR113808, GR125487, RS39604, SB204070 and a derivative thereof, as well as a pharmacologically acceptable salt thereof.

Piboserod is represented by the formula:

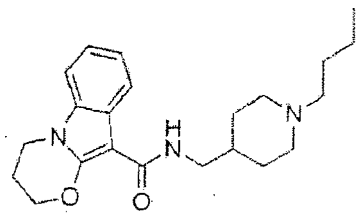

As used herein, the term "5-HT6 receptor antagonist" means a substance having the function of preventing serotonin from acting on a 5-HT6 receptor. Examples of the 5-HT6 receptor antagonist include cerlapirdine, clozapine and a derivative thereof, as well as a pharmacologically acceptable salt thereof.

Cerlapirdine is represented by the formula:

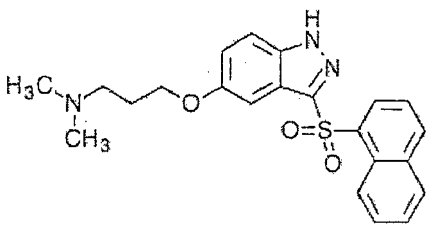

As used herein, the term "5-HT7 receptor antagonist" means a substance having the function of preventing serotonin from acting on a 5-HT7 receptor. Examples of the 5-HT7 receptor antagonist include lurasidone, metergoline, and a derivative thereof, as well as a pharmacologically acceptable salt thereof.

Metergoline is represented by the formula:

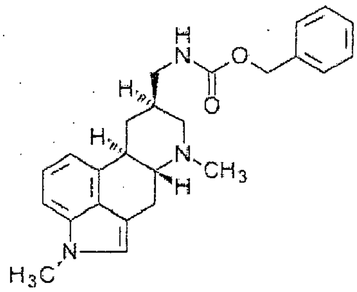

As used herein, the term "serotonin receptor agonist" means a substance having the function of acting on a serotonin receptor, and includes, for example, a 5-HT1 receptor agonist, and a 5-HT2 receptor agonist.

As used herein, the term "5-HT1 receptor agonist" means a substance having the function of acting on a 5-HT1 receptor. Examples of the 5-HT1 receptor agonist include piclozotan, tandospirone, sumatriptan, zolmitriptan, eletriptan, rizatriptan, naratriptan, almotriptan, frovatriptan, avitriptan, ergotamine, ergot alkaloid, and a derivative thereof, as well as a pharmacologically acceptable salt thereof.

Zolmitriptan is represented by the formula:

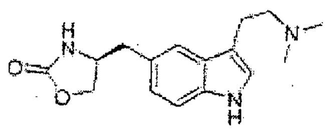

As used herein, the term "5-HT2 receptor agonist" means a substance having the function of acting on a 5-HT2 receptor. Examples of the 5-HT2 receptor agonist include α-methyl-5-HT, agomelatine, norfenfluramine, meta-chlorophenylpiperazine and a derivative thereof, as well as a pharmacologically acceptable salt thereof.

Agomelatine is represented by the formula:

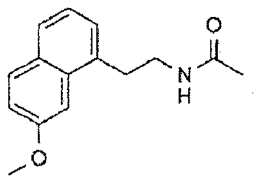

As used herein, the term "vasopressin receptor antagonist" means a substance having the function of preventing vasopressin from acting on a receptor, and includes, for example, a V2 receptor antagonist.

As used herein, the term "V2 receptor antagonist" means a substance having the function of preventing vasopressin from acting on a V2 receptor. Examples of the V2 receptor antagonist include tolvaptan, mozavaptan, conivaptan, lixivaptan, and a derivative thereof, as well as a pharmacologically acceptable salt thereof.

Mozavaptan is represented by the formula:

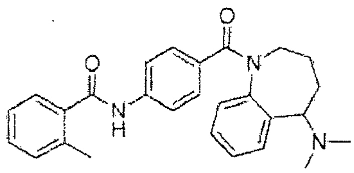

As used herein, the term "vasopressin receptor agonist" means a substance having the function of acting on a vasopressin receptor, and includes, for example, a V1 receptor agonist.

As used herein, the term "V1 receptor agonist" means a substance having the function of acting on a V1 receptor. Examples of the V1 receptor agonist include vasopressin, felypressin, desmopressin, lypressin, terlipressin, ornipressin, argipressin, and a derivative thereof, as well as a pharmacologically acceptable salt thereof.

Desmopressin is represented by the formula:

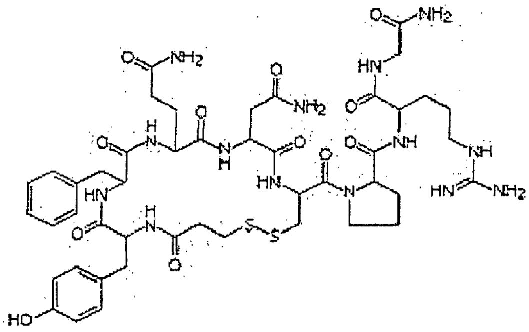

As used herein, the term "muscarine receptor antagonist" means a substance having the function of preventing acetylcholine from acting on a muscarine receptor, and includes, for example, a M1 receptor antagonist, a M3 receptor antagonist, and a M5 receptor antagonist.

As used herein, the term "M1 receptor antagonist" means a substance having the function of preventing acetylcholine from acting on a M1 receptor. The term "M3 receptor antagonist" means a substance having the function of preventing acetylcholine from acting on a M3 receptor. The term "M5 receptor antagonist" means a substance having the function of preventing acetylcholine from acting on a M5 receptor. Examples of the M1 receptor antagonist, and/or the M3 receptor antagonist, and/or the M5 receptor antagonist include pirenzepine, atropine, trimebutine, piperidolate, oxybutynin, tropicamide, propiverine, tolterodine, solifenacin, darifenacin, imidafenacin, oxyphencyclimine, tiotropium bromide, esoxybutynin, tiquizium, and a derivative thereof, as well as a pharmacologically acceptable salt thereof.

Oxybutynin is represented by the formula:

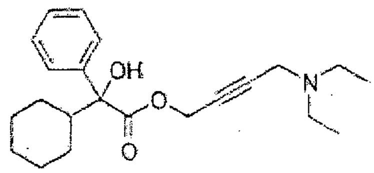

As used herein, the term "muscarine receptor agonist" means a substance having the function of acting on a muscarine receptor, and includes, for example, a M1 receptor agonist, a M2 receptor agonist, a M3 receptor agonist, a M4 receptor agonist, and a M5 receptor agonist.

As used herein, the term "M1 receptor agonist" means a substance having the function of acting on a M1 receptor. The term "M2 receptor agonist" means a substance having the function of acting on a M2 receptor. The term "M3 receptor agonist" means a substance having the function of acting on a M3 receptor. The term "M4 receptor agonist" means a substance having the function of acting on a M4 receptor. The term "M5 receptor agonist" means a substance having the function of acting on a M5 receptor. Examples of the M1 receptor agonist, and/or the M2 receptor agonist, and/or the M3 receptor agonist, and/or the M4 receptor agonist, and/or the M5 receptor agonist include acetylcholine, aceclidine, alvameline, talsaclidine, xanomeline, pilocarpine, cevimeline, bethanechol, mazaticol, muscarine, and a derivative thereof, as well as a pharmacologically acceptable salt thereof.

Bethanechol is represented by the formula:

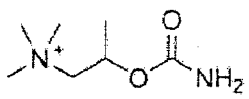

As used herein, the term "adrenalin receptor antagonist" means a substance having the function of preventing adrenalin from acting on a receptor, and includes, for example, an α1 receptor antagonist, a β1 receptor antagonist, a β2 receptor antagonist, and a β3 receptor antagonist.

As used herein, the term "α1 receptor antagonist" means a substance having the function of preventing adrenalin from acting on an α1 receptor. Examples of the α1 receptor antagonist include prazosin, doxazosin, bunazosin, trimazosin, alfuzosin, silodosin, terazosin, tamusulosin, and a derivative thereof, as well as a pharmacologically acceptable salt thereof.

Tamsulosin is represented by the formula:

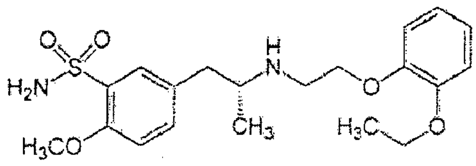

As used herein, the term "β1 receptor antagonist" means a substance having the function of preventing adrenalin from acting on a β1 receptor. The term "β2 receptor antagonist" means a substance having the function of preventing adrenalin from acting on a β2 receptor. The term "β3 receptor antagonist" means a substance having the function of preventing adrenalin from acting on a β3 receptor. Examples of the β1 receptor antagonist, and/or the β2 receptor antagonist, and/or the β3 receptor antagonist include bopindolol, pindolol, timolol, dichloroisoprenaline, alprenolol, carteolol, indenolol, bunitrclol, penbutolol, propranolol, nadolol, nipradilol, tilisolol, acebutolol, celiprolol, metoprolol, atenolol, bisoprolol, betaxolol, practolol, bevantolol, butoxamine, carvedilol, amosulalol, arotinolol, labetalol, and a derivative thereof, as well as a pharmacologically acceptable salt thereof.

Propranolol is represented by the formula:

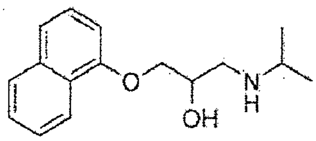

As used herein, the term "angiotensin receptor agonist" means a substance having the function of acting on an angiotensin receptor, and includes, for example, an AT2 receptor agonist.

As used herein, the term "adrenalin receptor agonist" means a substance having the function of acting on an adrenalin receptor, and includes, for example, an α1 receptor agonist, and an α2 receptor agonist.

As used herein, the term "α1 receptor agonist" means a substance having the function of acting on an α1 receptor. The term "α2 receptor agonist" means a substance having the function of acting on an α2 receptor. Examples of the α1 receptor agonist, and/or the α2 receptor agonist include norepinephrine, norfenefrine, etilefrine, naphazoline, phenylephrine, midodrine, methoxamine, oxedrine, metaraminol, arbutamine, ephedrine, oxymetazoline, tetryzoline, xylometazoline, tramazoline, pseudoephedrine, dipivefrine, amidephrine, methylephedrine, rilmenidine, brimonidine, medetomidine, xylazine, tizanidine, guanfacine, methyldopa, guanabenz, and a derivative thereof, as well as a pharmacologically acceptable salt thereof.

Xylazine is represented by the formula:

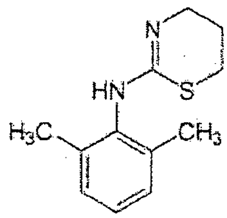

As used herein, the term "angiotensin receptor agonist" means a substance having the function of acting on an angiotensin receptor, and includes, for example, an AT2 receptor agonist.

As used herein, the term "AT2 receptor agonist" means a substance having the function of acting on an AT2 receptor. Examples of the AT2 receptor agonist include novokinin, angiotensin and a derivative thereof, as well as a pharmacologically acceptable salt thereof.

Angiotensin is represented by the formula:

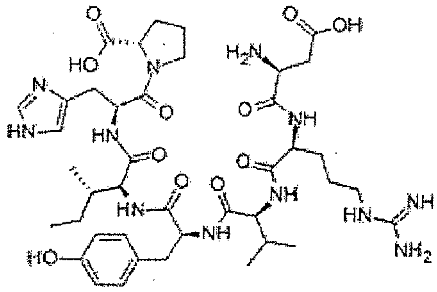

As used herein, the term "GABA receptor agonist" means a substance having the function of acting on a GABA receptor, and includes, for example, a $GABA_B$ receptor agonist.

As used herein, the term "$GABA_B$ receptor agonist" means a substance having the function of acting on a $GABA_B$ receptor. Examples of the $GABA_B$ receptor agonist include baclofen, γ-aminobutyric acid, arbaclofen and a derivative thereof, as well as a pharmacologically acceptable salt thereof.

Baclofen is represented by the formula:

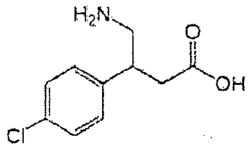

As used herein, the term "thrombin receptor antagonist" means a substance having the function of preventing thrombin from acting on a receptor, and includes, for example, a PAR-1 receptor antagonist.

As used herein, the term "PAR-1 receptor antagonist" means a substance having the function of preventing thrombin from acting on a PAR-1 receptor. Examples of the PAR-1 receptor antagonist include vorapaxar, atopaxar, FR171113, RWJ56110, dabigatran, dabigatran etexilate, melagatran, ximelagatran, hirudin, hirulog, argatroban and a derivative thereof, as well as a pharmacologically acceptable salt thereof.

Vorapaxar is represented by the formula:

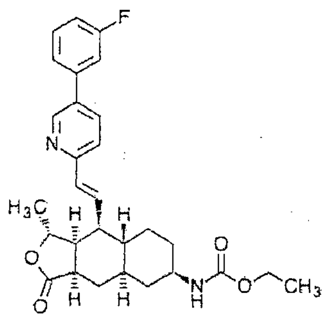

As used herein, the term "thrombin receptor agonist" means a substance having the function of acting on a thrombin receptor, and includes, for example, a PAR-1 receptor agonist.

As used herein, the term "PAR-1 receptor agonist" means a substance having the function of acting on a PAR-1 receptor. Examples of the PAR-1 receptor agonist include TRAP-6, TRAP-14, NAT6-$NH_2$ and a derivative thereof, as well as a pharmacologically acceptable salt thereof.

TRAP-6 is represented by the formula:

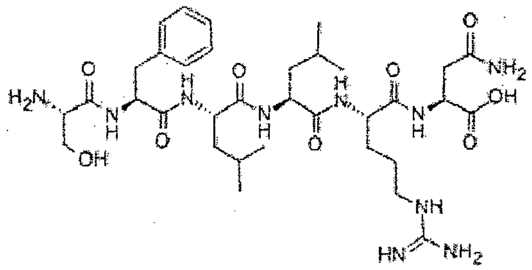

As used herein, the term "opioid receptor agonist" means a substance having the function of acting on an opioid receptor. Examples of the opioid receptor agonist include trimebutine, alvimopan, morphine, oxycodone, dihydrocodeine, diamorphine, pethidine, pentazocine, buprenorphine, butorphanol, nalbuphine, tilidine, dezocine, meptazinol, tapentadol, naltrexone, methadone, ethylmorphine, hydrocodone, acetyldihydrocodeine, nalorphine, loperamide, remoxipride, opipramol, and a derivative thereof, as well as a pharmacologically acceptable salt thereof.

Buprenorphine is represented by the formula:

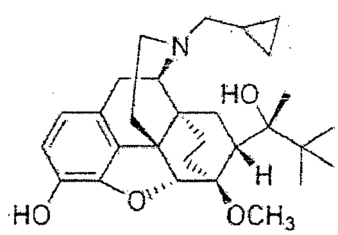

As used herein, the term "leukotriene receptor antagonist" means a substance having the function of preventing leukotriene from acting on a receptor, and includes, for example, a CysLT1 receptor antagonist, and a CysLT2 receptor antagonist.

As used herein, the term "CysLT1 receptor antagonist" means a substance having the function of preventing leukotriene from acting on a CysLT1 receptor. The term "CysLT2 receptor antagonist" means a substance having the function of preventing leukotriene from acting on a CysLT2 receptor. Examples of the CysLT1 receptor antagonist, and/or the CysLT2 receptor antagonist include montelukast, zafirlukast, pranlukast, and a derivative thereof, as well as a pharmacologically acceptable salt thereof. Examples of the pharmacologically acceptable salt of montelukast include montelukast sodium and the like.

Montelukast sodium is represented by the formula:

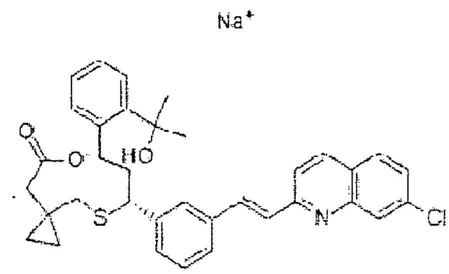

As used herein, the term "leukotriene receptor agonist" means a substance having the function of acting on a leukotriene receptor, and includes, for example, a BLT receptor agonist.

As used herein, the term "BLT receptor agonist" means a substance having the function of acting on a BLT receptor. Examples of the BLT receptor agonist include leukotriene B4, CAY10583 and a derivative thereof, as well as a pharmacologically acceptable salt thereof.

Leukotriene B4 is represented by the formula:

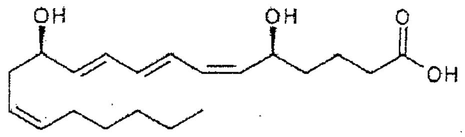

As used herein, the term "ADP receptor agonist" means a substance having the function of acting on an ADP receptor. Examples of the ADP receptor agonist include adenosine diphosphate, and a derivative thereof, as well as a pharmacologically acceptable salt thereof.

Adenosine diphosphate is represented by the formula:

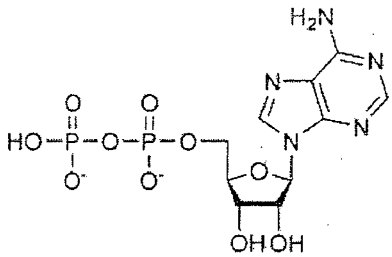

As used herein, the term "melatonin receptor agonist" means a substance having the function of acting on a melatonin receptor. Examples of the melatonin receptor agonist include melatonin, perlapine, tasimelteon, and a derivative thereof, as well as a pharmacologically acceptable salt thereof.

Melatonin is represented by the formula:

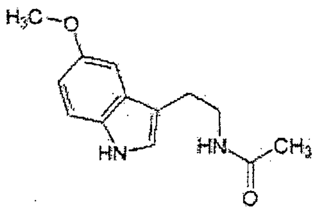

As used herein, the term "somatostatin receptor agonist" means a substance having the function of acting on a somatostatin receptor. Examples of the somatostatin receptor agonist include somatostatin, somatostatin-14, octreotide, and a derivative thereof, as well as a pharmacologically acceptable salt thereof.

Octreotide is represented by the formula:

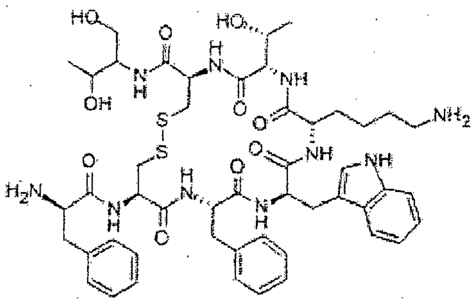

As used herein, the term "cannabinoid receptor agonist" means a substance having the function of acting on a cannabinoid receptor. Examples of the cannabinoid receptor agonist include dronabinol, nabilone, levonantradol, otenabant, GW833972A, GW405833, and a derivative thereof, as well as a pharmacologically acceptable salt thereof.

Dronabinol is represented by the formula:

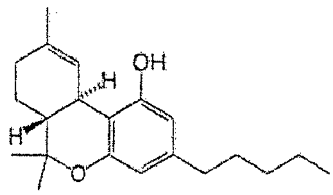

As used herein, the term "sphingosine-1 phosphate receptor agonist" means a substance having the function of acting on a sphingosine-1 phosphate receptor. Examples of the sphingosine-1 phosphate receptor agonist include fingolimod, ponesimod, RPC-1063, ONO-4641, SEW2871, sphingosine-1 phosphate and a derivative thereof, as well as a pharmacologically acceptable salt thereof.

Fingolimod is represented by the formula:

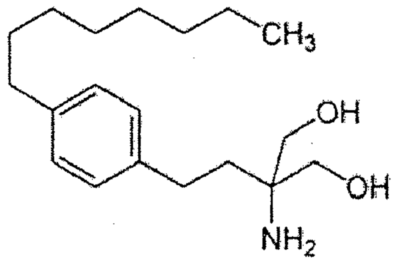

As used herein, the term "metabotropic glutamate receptor agonist" means a substance having the function of acting on a metabotropic glutamate receptor, and includes, for example, an mGluR2 receptor agonist, an mGluR3 receptor agonist, an mGluR4 receptor agonist, an mGluR6 receptor agonist, an mGluR7 receptor agonist, and an mGluR8 receptor agonist.

As used herein, the term "mGluR2 receptor agonist" means a substance having the function of acting on an mGluR2 receptor. The term "mGluR3 receptor agonist" means a substance having the function of acting on an mGluR3 receptor. The term "mGluR4 receptor agonist" means a substance having the function of acting on an mGluR4 receptor. The term "mGluR6 receptor agonist" means a substance having the function of acting on an mGluR6 receptor. The term "mGluR7 receptor agonist" means a substance having the function of acting on an mGluR7 receptor. The term "mGluR8 receptor agonist" means a substance having the function of acting on an mGluR8 receptor. Examples of the mGluR2 receptor agonist, and/or the mGluR3 receptor agonist, and/or the mGluR4 receptor agonist, and/or the mGluR6 receptor agonist, and/or the mGluR7 receptor agonist, and/or the mGluR8 receptor agonist include VU0361737, VU0155041, biphenylindanone A, PBDA, L-AP4, and a derivative thereof, as well as a pharmacologically acceptable salt thereof.

VU0361737 is represented by the formula:

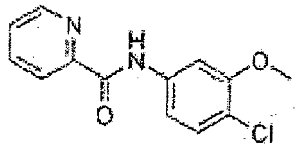

As used herein, the term "phospholipase A2 inhibitor" means a substance having the function of inhibiting the activity of phospholipase A2. Examples of the phospholipase A2 inhibitor include glycyrrhizic acid, glycyrrhetic acid, and a derivative thereof, as well as a pharmacologically acceptable salt thereof.

Glycyrrhetic acid is represented by the formula:

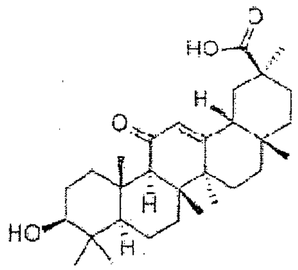

As used herein, the term "TGF-β production inhibitor" means a substance having the function of inhibiting production of TGF-β. Examples of the TGF-β production inhibitor include pirfenidone, tranilast, and a derivative thereof, as well as a pharmacologically acceptable salt thereof.

Pirfenidone is represented by the formula:

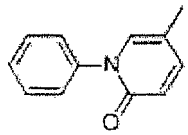

As used herein, the term "Th2 cytokine inhibitor" means a substance having the function of inhibiting production of a Th2 cytokine such as IL-4 and IL-5. Examples of the Th2 cytokine inhibitor include suplatast and a derivative thereof, as well as a pharmacologically acceptable salt thereof. Examples of the pharmacologically acceptable salt of suplatast include suplatast tosylate. In a preferable aspect of the present invention, the Th2 cytokine inhibitor is suplatast tosylate.

Suplatast tosilate is represented by the formula:

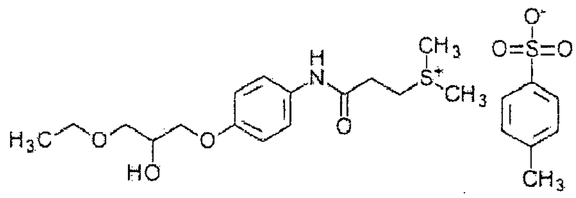

As used herein, the "pharmacologically acceptable acid" which can be contained in the composition of the present invention means an acid which has no harmful effect on a subject to which the composition is administered, and does not impair the pharmacological activity of the ingredients in the composition. In a preferable aspect of the present invention, the pharmacologically acceptable acid is an organic acid, more preferably an organic compound containing carboxyl group or an organic compound containing sulfonate group, more preferably a saturated or unsaturated straight or branched fatty acid in which the saturated straight chain part has 8 to 20 carbon atoms, lactic acid, malic acid, salicylic acid, maleic acid, citric acid, or an organic compound containing sulfonate group, more preferably a saturated or unsaturated straight or branched fatty acid in which the saturated straight chain part has 8 to 16 carbon atoms, lactic acid, malic acid, salicylic acid, maleic acid, citric acid, or an organic compound containing sulfonate group, still more preferably a fatty acid selected from the group consisting of decanoic acid, lauric acid, myristic acid, isostearic acid, palmitic acid, stearic acid and oleic acid, or lactic acid, salicylic acid, citric acid or methanesulfonic acid.

As used herein, a "pharmacologically acceptable salt" which can be contained in the composition of the present invention means a salt which has no harmful effect on an the subject to be administered with the composition, and does not impair the pharmacological activity of the ingredients in the composition. Examples of pharmacologically acceptable salts include inorganic acid salts (e.g. hydrochloric acid salt and phosphoric acid salt), organic acid salts (e. g. acetic acid salt, phthalic acid salt, and TFA salt), metal salts (alkali metal salts (e.g. sodium salt and potassium salt), alkaline earth metal salts (e.g. calcium salt and magnesium salt), aluminum salt etc.), and amine salts (triethylamine salt, benzylamine salt, diethanolamine salt, t-butylamine salt, dicyclohexylamine salt, arginine salt, dimethylammonium salt, ammonium salt etc.), but are not limited to them.

As used herein, the term "immunomodulatory small molecule drug" means a substance which activates or suppresses immune cells such as a T cell, a NK cell, a macrophage and the like, and which does not correspond to any of the aforementioned TLR ligand, cyclic dinucleotide, helper peptide, cyclooxygenase inhibitor, prostaglandin receptor antagonist, prostaglandin receptor agonist, TSLP production inhibitor, adenylate cyclase inhibitor, omega-3 fatty acid,

PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-β production inhibitor, and Th2 cytokine inhibitor. Examples of the immunomodulatory small molecule drug include bestatin, pidotimod, levamisole, golotimod, forphenicinol, and a derivative thereof, as well as a pharmacologically acceptable salt thereof. Examples of the pharmacologically acceptable salt of levamisole include levamisole hydrochloride.

Bestatin is represented by the formula:

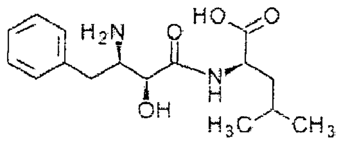

Pidotimod is represented by the formula:

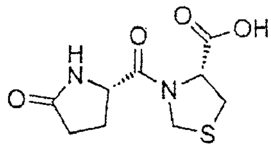

Levamisole hydrochloride is represented by the formula:

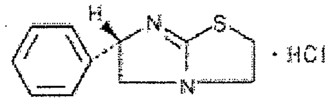

In the present invention, the immunomodulatory small molecule drug is usually a compound having a molecular weight of less than 1000, preferably less than 500. In a preferable aspect of the present invention, the immunomodulatory small molecule drug is one or more compounds selected from the group consisting of bestatin, pidotimod and levamisole hydrochloride.

As above described, the inventors have found that among a variety of cellular immunity induction promoters, a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenalin receptor antagonist, an adrenalin receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, and a Th2 cytokine inhibitor are particularly suitable for enhancing immune response induced by transdermal administration of the HER2/neu E75 peptide and/or the modified HER2/neu E75 peptide. Therefore, in one aspect, the cellular immunity induction promoter of the present invention is one or more substances selected from them. In further aspect, such a substance is a first cellular immunity induction promoter. In particularly preferred aspect of the present invention, the cellular immunity induction promoter is a combination of one or more substance selected from a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an

adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor and a Th2 cytokine inhibitor, and a helper peptide. The induction of cellular immunity can be determined quantitatively by a variety of known methods. Any of those known method, for example, the ELISPOT method described in Examples may be used.

As used herein, the non-invasive administration means administration without actively giving physical irritation and/or chemical irritation, preferably without giving physical irritation (e.g. without giving irritation by tape stripping or microneedle) to a skin.

As used herein, the term "mildly irritating condition" means a condition under which irritation to be given to the skin is lower than the irritation generally given in order to improve the skin permeability of the antigen contained in conventional vaccines, or a condition under which irritation is not given to the skin at all. In general, physical and/or chemical stimulation is given to the skin before or simultaneously with the transdermal administration of a conventional vaccine composition so that the antigen can penetrate through the skin. In a preferable aspect of this invention, examples of the mildly irritating condition include a condition of low physical irritation and a condition of low chemical irritation. The condition of low physical irritation is, for example, a condition under which transepidermal water loss (TEWL) ($g/h{\cdot}m^2$) in the model animal for skin irritation evaluation is 50 or less, preferably 45 or less, more preferably 40 or less, even more preferably 35 or less, further preferably 30 or less. Since the TEWL level in non-treated skin is about 2 ($g/h{\cdot}m^2$), the TEWL level before the administration of the vaccine composition may be 2 ($g/h{\cdot}m^2$) or more. The condition of low chemical irritation is, for example, a condition under which the thymic stromal lymphopoietin (TSLP) level (pg/mg protein) in the skin of the model animal for skin irritation evaluation is 10000 or less, preferably 9000 or less, more preferably 8000 or less, further preferably 7000 or less. Since the TSLP level is about 1 (pg/mg protein) in non-treated skin, the TSLP level at completion of the administration of the vaccine composition exceeds 1 (pg/mg protein), preferably exceeds 2 (pg/mg protein), more preferably exceeds 3 (pg/mg protein). The "thymic stromal lymphopoietin (TSLP) " is a cytokine which participates in differentiation and recruitment of T cells, and can be utilized as an index of the degree of skin irritation in the present invention. Greater TSLP value means stronger skin irritation. Examples of means for attaining the condition of low physical irritation include not-conducting the conventional pre-treatment of the skin before the administration such as not conducting tape stripping or microneedle puncture before the administration. Examples of means for attaining the condition of low chemical irritation include avoiding administration of an irritating chemical ingredient such as ethanol or a surfactant at a certain amount or more. The procedure for attaining the mildly irritating condition can be determined by using a model animal for skin irritation evaluation, and the determined procedure can be applied to the subject to be treated by the vaccine composition, for example, a human subject.

As used herein, the term "cancer" means an abnormal expression of HER2/neu gene, for example, a cancer accompanying an overexpression, for example, an adenocarcinoma. The adenocarcinoma accompanying the overexpression of HER2/neu gene includes breast cancer or prostate cancer, without limitation.

As used herein, the term "abnormal expression of a gene" means that the expression level of the gene in a cell is increased or decreased dramatically, for example, two times or more, for example, 4 times or more, compared to other cells in the same cell. The term "overexpression" means that the abnormal expression is an increase of the expression level. The expression level of the gene can easily be measured by any well-known method in the art.

As used herein, the term "subject" means any animal having the HER2/neu gene, immune response of which can be induced by the transdermal administration of a cancer vaccine composition for transdermal administration at a practical stage. Typically, the subject may be a mammal such as human, mouse, rat, dog, cat, rabbit, horse, cow, sheep, pig, goat, monkey, and chimpanzee. A particularly preferable subject is human.

As used herein, the term "model animal for immunological evaluation" means a model animal for evaluating the property of a cancer vaccine composition for transdermal administration to induce immunity. Specifically, it means a model animal for evaluating the property of inducing cellular immunity. The model animal for immunological evaluation is selected in view of compatibility between the antigen in the vaccine composition to be evaluated and the MHC class 1 molecule of the animal. For example, in the case of a vaccine composition containing a HLA-A*24 type MHC restricted class 1 peptide, the property may be evaluated in a BALB/c mouse. In the case of a vaccine composition containing a HLA-A*02 type MHC restricted peptide, the property may be evaluated in a genetically modified mouse by which cellular immunity induction by the HLA-A*02 type MHC restricted peptide can be evaluated. In the case of a vaccine composition containing other HLA type MHC restricted peptide, the property is evaluated in an animal by which cellular immunity induction by the HLA type MHC restricted peptide can be evaluated. In the case of a vaccine composition containing a protein antigen, the property is evaluated in an animal having MHC compatible with a class 1 epitope to be used to induce the cellular immunity, among various class 1 epitopes included in the amino acid sequence of the protein antigen. When the hair of the animal is cut to ensure the place for transdermal administration, the animal should be used after it is recovered from the skin damage caused by the hair cut.

As used herein, the term "model animal for skin irritation evaluation" means a model animal for evaluating transepidermal water loss (TEWL) as an index of physical irritation of the skin, or a model animal for evaluating TSLP as an index of the skin irritation property of a cancer vaccine composition for transdermal administration. Regardless of the kind of the antigen contained in the cancer vaccine composition for transdermal administration, C57BL/6 mouse may be used as model animal for skin irritation evaluation. When the hair of the animal is cut to ensure the place for the transdermal administration, the animal should be used after it is recovered from the skin damage caused by the hair cut.

II. Cancer Vaccine composition for Transdermal Administration

The usefulness of the HER2/neu E75 peptide as a cancer vaccine has been already apparent (Non-Patent Document 3, Non-Patent Document 4).

As used herein, the composition "for transdermal administration" may be provided in any formulation or preparation which is usually used in the transdermal administration, for example, a liquid formulation for external use such as a liniment formulation or a lotion formulation, a spray formulation for external use such as an aerosol formulation, an ointment formulation, a plaster formulation, a cream formulation, a gel formulation, or a patch preparation such as a tape preparation or a cataplasm preparation. Grouping, definition, a nature, a production process and the like of these formulations and preparations are well-known in the art. For example, see Japanese Pharmacopoeia 16th edition.

Examples of the base for liniment formulation include water, ethanol, fatty oils such as hard paraffin, soft paraffin, liquid paraffin, glycerin, paraffin oil and beeswax, metal soap; mucilages; natural oils (e.g. almond oil, corn oil, peanut oil, castor oil, olive oil, or a derivative thereof (such as polyoxyl castor oil)); mutton tallow or a derivative thereof, fatty acids and/or esters (e.g. stearic acid, oleic acid, isopropyl myristate).

The lotion formulation is a preparation in which the active ingredient is finely and homogeneously dispersed in an aqueous liquid, and there are a suspending lotion formulation and an emulsified lotion formulation. Examples of the suspending agent include gum arabic, sodium alginate, carboxymethylcellulose sodium, methylcellulose, bentonite and the like. Examples of the emulsifying agent include sodium lauryl sulfate, sorbitan fatty acid ester and the like.

For example, as an ointment base, hydrophobic base such as fats or oils, waxes and hydrocarbon compounds can be generally used. Specifically, examples of the ointment base include mineral bases such as yellow vaseline, white vaseline, paraffin, liquid paraffin, plastibase, silicone and the like, and animal or plant bases such as beeswax, animal or vegetable fat or oil and the like.

Examples of the base for cream formulation include water/oil type bases such as hydrophilic ointment, vanishing cream and the like; and oil/water type bases such as hydrophilic vaseline, purified lanolin, Aquahole, Eucerin, Neocerin, hydrous lanolin, cold cream, hydrophilic plastibase and the like.

As a gel base, for example, a carboxyvinyl polymer, a gel base, a fat-free ointment, polyvinylpyrrolidone, polyvinyl alcohol, sodium polyacrylate, carboxymethylcellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, a carboxyvinyl polymer, tragacanth, gum arabic, tara gum, tamarind seed gum, psyllium seed gum, agar, gellan gum, glucomannan, locust bean gum, guar gum, carrageenan, dextrin, dextran, amylose, carboxymethylcellulose potassium, carboxymethylcellulose sodium, carboxymethylcellulose calcium, pullulan, chitosan, sodium carboxymethyl starch, plantago testa, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate/methacrylic acid/methyl methacrylate copolymer, ethyl acrylate/methyl methacrylate copolymer, polyvinylacetal diethylaminoacetate, casein, alginic acid alkyl ester, gelatin, polyethylene glycol and the like as a hydrogel base can be used.

Examples of the base for a cataplasm preparation include gelatin, carboxymethylcellulose sodium, methylcellulose, sodium polyacrylate, kaolin, polyvinyl alcohol, polyvinylpyrrolidone, glycerin, propylene glycol, water and the like.

For example, a tape preparation comprises an adhesive layer comprising an acrylic adhesive, a natural rubber adhesive, a synthetic rubber adhesive (including rubber elastomer such as synthetic isoprene rubber, polyisobutylene (PIB), styrene-butadiene rubber, styrene-isoprene-styrene (SIS) rubber etc.), a silicone adhesive, a vinyl ester adhesive, a vinyl ether adhesive or the like, and a support which supports the adhesive layer. Optionally, the preparation may further contain a release liner which covers the adhesive layer to avoid exposure thereof before use and can easily be peeled from the adhesive layer upon use.

The amount of the HER2/neu E75 peptides and/or the modified HER2/neu E75 peptide and the cellular immunity induction promoter in the cancer vaccine composition of the present invention is not particularly limited. In one aspect, the cancer vaccine composition of the present invention contains the HER2/neu E75 peptides and/or the modified HER2/neu E75 peptide preferably in an amount of 0.01 to 40% by weight, more preferably 0.1 to 30% by weight based on the total weight of the composition. In one aspect, the cancer vaccine composition of the present invention contains the cellular immunity induction promoter preferably in an amount of 0.001 to 30% by weight, more preferably 0.01 to 20% by weight based on the total weight of the composition.

When the composition of the present invention is provided in the form of a tape preparation, the tape preparation contains an adhesive layer containing the active ingredient which is the HER2/neu E75 peptides and/or the modified HER2/neu E75 peptide, and a support which supports the adhesive layer, in the case of a matrix type tape preparation. In the case of a reservoir type tape preparation, the tape preparation contains a reservoir containing the active ingredient and an adhesive layer, and a support which supports the reservoir and an adhesive layer. The tape preparation, as described below, optionally, may further contain a release liner which does not expose the adhesive layer before use and can be easily peeled from the adhesive layer upon use. Hereinafter, representatively, a tape preparation will be explained in detail. The adhesive layer of the tape preparation (hereinafter, also referred to as "tape preparation of the present invention") contains the HER2/neu E75 peptides and/or the modified HER2/neu E75 peptide, and the cellular immunity induction promoter. In one aspect, the adhesive layer of the tape preparation of the present invention contains the HER2/neu E75 peptides and/or the modified HER2/neu E75 peptide preferably in an amount of 0.01 to 40% by weight, more preferably 0.1 to 30% by weight based on the total weight of the adhesive layer. In one aspect, the adhesive layer of the tape preparation of the present invention contains the cellular immunity induction promoter preferably in an amount of 0.001 to 30% by weight, more preferably 0.01 to 20% by weight based on the total weight of the adhesive layer.

An adhesive which is to form the adhesive layer of the tape preparation of the present invention is not particularly limited, and examples thereof include acrylic adhesives consisting of an acrylic polymer; rubber adhesives comprising a rubber elastomer such as a styrene-diene-styrene block copolymer (e.g. styrene-isoprene-styrene block copolymer, styrene-butadiene-styrene block copolymer etc.), polyisoprene, polyisobutylene, butyl rubber, polybutadiene and the like; silicone adhesives such as silicone rubber, dimethylsiloxane base, diphenylsiloxane base and the like; vinyl ether adhesives such as polyvinyl methyl ether, polyvinyl ethyl ether, polyvinyl isobutyl ether and the like; vinyl ester adhesives such as vinyl acetate-ethylene copolymer and the like; and polyester adhesives consisting of a carboxylic acid component such as dimethyl terephthalate, dimethyl isophthalate, dimethyl phthalate, and a polyhydric alcohol component such as ethylene glycol. A particularly preferable adhesive is an acrylic adhesive, a rubber adhesive, and a silicone adhesive. These adhesives are contained in the adhesive layer preferably in an amount of 10 to 90% by weight, more preferably 20 to 80% by weight, as a solid matter thereof, based on the total weight of the adhesive layer.

Examples of the acrylic adhesive include an acrylic acid ester adhesive containing, as a main component, a polymer comprising (meth)acrylic acid C2-C18 alkyl ester as a first monomer. Examples of the (meth) acrylic acid alkyl ester (first monomer) include (meth)acrylic acid alkyl esters in which an alkyl group is a straight, branched or cyclic alkyl group having 1 to 18 carbon atoms (e.g. methyl, ethyl, propyl, butyl, pentyl, hexyl, cyclohexyl, heptyl, octyl, 2-ethylhexyl, nonyl, decyl, undecyl, dodecyl, tridecyl etc.). Preferred are (meth)acrylic acid alkyl esters in which an alkyl group is a straight, branched or cyclic alkyl group having 4 to 18 carbon atoms (e.g. butyl, pentyl, hexyl, cyclohexyl, heptyl, octyl, 2-ethylhexyl, nonyl, decyl, undecyl, dodecyl, tridecyl etc.). Further, since use of a monomer component which lowers the glass transition temperature of a polymer is suitable in order to impart adhesiveness at a room temperature, (meth)acrylic acid alkyl esters in which an alkyl group is a straight, branched or cyclic alkyl group having 4 to 8 carbon atoms (e.g. butyl, pentyl, hexyl, cyclohexyl, heptyl, octyl, 2-ethylhexyl etc. , preferably butyl, 2-ethylhexyl, and cyclohexyl, particularly preferably 2-ethylhexyl) are more preferable. Specifically, butyl acrylate, 2-ethylhexyl acrylate, 2-ethylhexyl methacrylate, cyclohexyl acrylate, cyclohexyl methacrylate and the like are more preferable and, among them, 2-ethylhexyl acrylate is most preferable. These (meth)acrylic acid alkyl esters (first monomer component) can be used alone, or can be used by combining two or more thereof.

In addition, the acrylic adhesive may contain a second monomer copolymerizable with the (meth) acrylic acid alkyl ester, and examples of the second monomer include monomers having a functional group which can become a crosslinking point upon use of a crosslinking agent. Examples of the functional group which can participate in a crosslinking reaction include a hydroxy group, a carboxyl group, a vinyl group and the like, and a hydroxy group and a carboxyl group are preferable. Specific examples of the monomer (second monomer component) include (meth) acrylic acid hydroxyethyl ester, (meth)acrylic acid hydroxypropyl ester, N-hydroxyalkyl(meth)acrylamide, (meth) acrylic acid, itaconic acid, maleic acid, maleic anhydride, mesaconic acid, citraconic acid, glutaconic acid and the like. Among them, from the viewpoint of easy availability, acrylic acid, methacrylic acid, and acrylic acid hydroxyethyl ester (particularly, 2-hydroxyethyl acrylate) are preferable, and acrylic acid is most preferable. These monomers (second monomer component) can be used alone, or can be used by combining two or more thereof.

Further, the acrylic adhesive may optionally contain a third monomer in addition to the second monomer. Examples of the third monomer (third monomer component) include vinyl esters such as vinyl acetate, vinyl propionate and the like; vinyl ethers such as methyl vinyl ether, ethyl vinyl ether and the like; vinyl amides such as N-vinyl-2-pyrrolidone, N-vinylcaprolactam and the like; (meth)acrylic acid alkoxy esters such as (meth)acrylic acid methoxyethyl ester, (meth)acrylic acid ethoxyethyl ester, (meth)acrylic acid tetrahydrofurfuryl ester and the like; hydroxy group-containing monomers (since this is used as a third monomer component, it is not a crosslinking point) such as hydroxypropyl (meth)acrylate, $\alpha$-hydroxymethyl acrylate and the like; (meth)acrylic acid derivatives having an amide group such as (meth)acrylamide, dimethyl(meth)acrylamide, N-butyl(meth)acrylamide, N-methylol(meth)acrylamide and the like; (meth)acrylic acid aminoalkyl esters such as (meth)acrylic acid aminoethyl ester, (meth)acrylic acid dimethylaminoethyl

ester, (meth)acrylic acid t-butylaminoethyl ester and the like; (meth)acrylic acid alkoxyalkylene glycol esters such as (meth)acrylic acid methoxyethylene glycol ester, (meth)acrylic acid methoxydiethylene glycol ester, (meth)acrylic acid methoxypolyethylene glycol ester, (meth)acrylic acid methoxypolypropylene glycol ester and the like; (meth)acrylonitriles; monomers having sulfonic acid such as styrenesulfonic acid, allylsulfonic acid, sulfopropyl(meth)acrylate, (meth)acryloyloxynaphthalenesulfonic acid, acrylamidemethylsulfonic acid and the like; and vinyl-group-containing monomers such as vinylpiperidone, vinylpyrimidine, vinylpiperazine, vinylpyrrole, vinylimidazole, vinyloxazole, vinylmorpholine and the like. Among them, vinyl esters and vinyl amides are preferable, vinyl acetate is preferable as vinyl esters, and N-vinyl-2-pyrrolidone is preferable as vinyl amides. These monomers (third monomer component) can be used alone, or can be used by combining two or more kinds thereof.

When the acrylic adhesive is a copolymer of a (meth)acrylic acid alkyl ester (first monomer component) and a vinyl monomer having a functional group which can participate in a crosslinking reaction (second monomer component), the (meth)acrylic acid alkyl ester and the vinyl monomer having a functional group which can participate in a crosslinking reaction are copolymerized by blending the components at a weight ratio of (meth) acrylic acid alkyl ester : vinyl monomer having a functional group which can participate in a crosslinking reaction of preferably 99 to 85 : 1 to 15, more preferably 99 to 90 : 1 to 10.

Further, when the acrylic adhesive is a copolymer of a (meth)acrylic acid alkyl ester (first monomer component), a vinyl monomer having a functional group which can participate in a crosslinking reaction (second monomer component), and a monomer other than them (third monomer component), the (meth)acrylic acid alkyl ester, the vinyl monomer having a functional group which can participate in a crosslinking reaction, and the monomer other than them are copolymerized by blending the components at a weight ratio of (meth) acrylic acid alkyl ester : vinyl monomer having a functional group which can participate in a crosslinking reaction : monomer other than them of preferably 40 to 94 : 1 to 15 : 5 to 50, more preferably 50 to 89 : 1 to 10 : 10 to 40.

Polymerization reaction may be performed by any known method, and is not particularly limited. For example, the monomers in a solvent such as ethyl acetate may be reacted in the presence of a polymerization initiator (e.g. benzoyl peroxide, azobisisobutyronitrile etc.) at 50 to 70°C for 5 to 48 hours.

Particularly preferable acrylic adhesives in the present invention are, for example, a copolymer of acrylic acid 2-ethylhexyl ester/acrylic acid/N-vinyl-2-pyrrolidone, a copolymer of acrylic acid 2-ethylhexyl ester/N-(2-hydroxyethyl)acrylamide/N-vinyl-2-pyrrolidone, a copolymer of acrylic acid 2-ethylhexyl ester/acrylic acid 2-hydroxyethyl ester/vinyl acetate, and a copolymer of acrylic acid 2-ethylhexyl ester/acrylic acid, and more preferably, a copolymer of acrylic acid 2-ethylhexyl ester/acrylic acid/N-vinyl-2-pyrrolidone.

Optionally, these acrylic adhesives may be subjected to physical crosslinking treatment by ultraviolet irradiation, or radiation irradiation such as electron beam irradiation, or chemical crosslinking treatment using various crosslinking agents such as an isocyanate compound such as trifunctional isocyanate, organic peroxide, organic metal salt, metal alcoholate, metal chelate compound, polyfunctional compound (polyfunctional external crosslinking agent, a monomer for polyfunctional internal crosslinking such as diacrylate and dimethacrylate).

Examples of the rubber adhesive include rubber adhesives in whicha rubber elastomer such as polyisobutylene/polybutene elastomer, styrene/diene/styrene block copolymer, styrene/butadiene elastomer, nitrile elastomer, chloroprene elastomer, vinylpyridine elastomer, polyisobutylene elastomer, butyl elastomer, or isoprene/isobutylene elastomer is blended. Among them, in view of solubility of the peptide and the cellular immunity induction promoter in the adhesive and the skin adhesiveness, polyisobutylene (PIB), styrene/diene/styrene block copolymer (e.g. styrene/butadiene/styrene block copolymer (SBS), styrene/isoprene/styrene block copolymer (SIS) etc.) and the like are preferably used. A mixture of two or more of those adhesives may also be used.

Further, in order to achieve a suitable adhesive force and drug solubility of the rubber adhesive, the rubber adhesive may be a mixture of two or more rubber elastomers of the same or different monomer components each having different average molecular weights. For example, with respect to polyisobutylene, a mixture of polyisobutylene of high molecular weight having an average molecular weight of 150,000 to 5,500,000, polyisobutylene of medium molecular weight having an average molecular weight of 10,000 to 150,000 and/or polyisobutylene of low molecular weight having an average molecular weight of 500 to 4,000 is preferable. In this case, it is preferable to blend polyisobutylenes of high molecular weight, medium molecular weight and low molecular weight at a weight ratio of high molecular weight : medium molecular weight : low molecular weight = 10 to 80, preferably 20 to 70 : 0 to 90, preferably 10 to 80 : 0 to 80, preferably 10 to 60.

As used herein, the average molecular weight means a viscosity average molecular weight calculated from Flory viscosity equation. The average molecular weight is calculated by calculating Staudinger index ($J_0$) by Schulz-Blaschke equation from a flow time of a capillary 1 on an Ubbelohde type viscometer at 20°C, and then using the $J_0$ value in the following expression.

(Formula)

$$J_0 = \eta_{sp}/c(1 + 0.31\eta_{sp}) \text{ (Schulz-Blaschke equation)}$$

$$\eta_{sp} = t/t_0 - 1$$

t: Flow time of solution (according to Hagenbach-couette correction formula)
$t_0$: Flow time of solvent (according to Hagenbach-couette correction formula)
c: Concentration of solution (g/cm$^3$)

$$J_0 = 3.06 \times 10^{-2}\ \overline{Mv}^{\,0.65}$$

$\overline{Mv}$: Viscosity average molecular weight

In order to impart suitable adhesiveness, for example, a tackifier such as a rosin resin, a polyterpene resin, a coumarone-indene resin, a petroleum resin, a terpene-phenol resin, a xylene resin, an alicyclic saturated hydrocarbon resin or the like may be blended in the rubber adhesive. One, two or more kinds of tackifiers can be blended in an amount of 50% by weight or less, preferably 5 to 40% by weight based on the total weight of the rubber adhesive.

Examples of the silicone adhesive include silicone adhesives consisting of polyorganosiloxane adhesive, polydimethylsiloxane adhesive, and polydimethyldiphenyl-siloxane adhesive. Inter alia, a commercially available silicone adhesive such as BIO PSA from Dow Corning Corporation is preferably used.

The support which supports the adhesive layer is not particularly limited, and a support that is substantially impervious to the peptide and the cellular immunity induction promoter so that the peptide, the cellular immunity induction promoter, additives or the like contained in the adhesive layer will not pass through the support to leak from the rear surface.

As the support, for example, a single film of polyester, polyamide, poly(vinylidene chloride), polyethylene, polypropylene, poly(vinyl chloride), ethylene-ethyl acrylate copolymer, polytetrafluoroethylene, ionomer resin, metal foil or the like, or a laminate film of them can be used. Among them, in order to make adhesiveness (anchorability) between the support and the adhesive layer good, it is preferable that the support is a laminate film of a nonporous plastic film and a porous film made of the aforementioned material. In this case, it is desirable that the adhesive layer is formed on the porous film side. As such a porous film, a porous film which improves anchorability with the adhesive layer is adopted, and specific examples thereof include a paper sheet, a woven fabric, a non-woven fabric, a knitted fabric, a sheet which has been mechanically perforation-treated, and the like. Among them, from the viewpoint of handling property and the like, particularly, a paper sheet, a woven fabric and a non-woven fabric are preferable. As the porous film, in view of improvement in anchorability, softness and sticking operability of a tape preparation and the like, a porous film having a thickness in the range of 1 to 200 $\mu$m is adopted. In addition, when a woven fabric or a non-woven fabric is used as the porous film, the weight per unit area is preferably 5 to 30 g/m$^2$, more preferably 6 to 15 g/m$^2$.

Examples of the most suitable support include a laminate film composed of a polyester film (preferably, polyethylene terephthalate film) having a thickness of 1.5-6 $\mu$m and a non-woven fabric made by a polyester (preferably, polyethylene terephthalate) having a weight per unit area of 6-15 g/m$^2$.

In the tape preparation of the present invention, in order to protect the surface of the adhesive layer until use, it is desirable that a release liner is laminated on the adhesive surface. The release liner is not particularly limited as far as it is treated so that it has the releasing property and it can be released with a sufficiently small peeling force. For example, a film of polyester, poly(vinyl chloride), poly(vinylidene chloride), polyethylene terephthalate or the like, paper such as pure paper, glassine paper and the like, or a laminate film of pure paper or glassine paper and polyolefin may be treated by coating a silicone resin, a fluorine resin or the like on the surface to be contacted with the adhesive layer and is used as the release liner. The thickness of the release liner is preferably 10 to 200 $\mu$m, more preferably 25 to 100 $\mu$m. As the release liner, polyester layer, particularly, polyethylene terephthalate layer is preferable in view of the barrier property and the cost. Further, in this case, in terms of handling property, a release liner having a thickness of around 25 to 100 $\mu$m is preferable.In the tape preparation of the invention, a release liner is preferably laminated to the adhesive face of the adhesive layer in order to protect the adhesive face before use. The release liner is not particularly limited, as long as the liner has a sufficiently low release force by releasing process. For example, used are films such as polyester, polyvinylchloride, polyvinylidene chloride, polyethylene terephthalate; papers such as high quality paper or glassine paper; or laminated films composed of a quality paper or glassine paper and polyolefin, which are treated by releasing process by applying silicone resins or fluorine resins on the contact surface to the adhesive layer. The

release liner preferably has a thickness of 10-200 μm, more preferably 25-100 μm. The release liner is preferably made by polyester (in particular, polyethylene terephthalate) resin in view of barrier property, cost, and the like. Further, in this case, the liner preferably has a thickness 25-100 μm in view of handling property.

Further, the composition of the present invention may contain an additive, if necessary. The additive is selected from, for example, isotonizing agents, antiseptics/germicides, antioxidants, resolvents, solubilizers, suspending agents, fillers, pH adjusting agents, stabilizers, absorption promoters, release rate controlling agents, coloring agents, plasticizers, crosslinking agents, adhesives and the like, or a combination of two or more kinds of them, depending on the compatibility with the main ingredient of the base, the HER2/neu E75 peptide and/or the modified HER2/neu E75 peptide and the cellular immunity induction promoter, intended administration regimen and the like. In addition, when the composition of the present invention is in a form of a tape preparation, the tape preparation can contain a skin permeability enhancer as an additive.

As used herein, the term "skin permeability enhancer" means any substance which can improve an efficiency of permeation of a transdermally administered antigen through the skin, as compared with the efficiency obtained without the substance. The skin permeability enhancer is not particularly limited as far as it is liquid at room temperature (25°C), that is, has fluidity at that temperature and has an absorption promoting effect. When the skin permeability enhancer is a mixture of two or more substances, the mixture is liquid at room temperature (25°C) and has an absorption promoting effect. The skin permeability enhancer may be an organic liquid and preferably, a hydrophobic liquid in view of their compatibility with the adhesive layer.

Examples of skin permeability enhancers include higher alcohols such as oleyl alcohol, octyldodecanol and the like; polyhydric alcohols such as glycerin, ethylene glycol, polypropylene glycol and the like; higher fatty acids such as oleic acid, caprylic acid and the like; fatty acid esters such as isopropyl myristate, isopropyl palmitate, ethyl oleate and the like; polybasic acid esters such as diethyl sebacate, diisopropyl adipate and the like; polyhydric alcohol fatty acid esters such as diglyceryl triisostearate, monooleic acid sorbitan, dicaprylic acid propylene glycol, monolauric acid polyethylene glycol, tetraoleic acid polyoxyethylene sorbit and the like; polyoxyethylene alkyl ethers such as polyoxyethylene lauryl ether and the like; hydrocarbons such as squalane, liquid paraffin and the like; plant oils such as olive oil, castor oil and the like; silicone oils; pyrrolidones such as N-methylpyrrolidone, N-dodecylpyrrolidone and the like; sulfoxides such as decylmethyl sulfoxide and the like, and these can be used alone, or can be used by mixing two or more kinds thereof.

When rubber adhesives or acrylic adhesives are used, a second skin permeability enhancer can be used. Specific example of the second skin permeability enhancer includes polyvinylpyrrolidone, crospovidone, polypropylene glycol, polyvinyl alcohol, carboxy vinyl polymer, hydroxypropylcellulose, or mixture thereof, without limitation. In a preferred aspect, the second skin permeability enhancer of the invention is polyvinylpyrrolidone, crospovidone and/or polypropylene glycol.

In view of improving skin permeability of the HER2/neu E75 peptide, the skin permeability enhancer which is preferably used includes higher alcohols, more specifically, higher alcohols having a carbon number of 8-18 (preferably 8-14), fatty acid esters, more specifically, fatty acid esters of a fatty acid having a carbon number of 8-18 (preferably 12-16) and a monovalent alcohol having a carbon number of 1-18, polyhydric alcohol fatty acid esters, in particular, fatty acid esters, in particular, isopropyl myristate, isopropyl palmitate, or diethyl sebacate. The amount of the skin permeability enhancer is preferably from 0.1% by weight to 70% by weight, more preferably from 1% by weight to 65% by weight, more preferably from 5% by weight to 60% by weight based on a total amount of the adhesive layer. When the amount of the skin permeability enhancer is 0.1% by weight or more, high transdermal proabsorptive effect is obtained. When the amount of the skin permeability enhancer is 70% by weight or less, high transdermal proabsorptive effect is advantageously obtained while preventing the decrease of total adhesiveness and cohesion force of the adhesive layer.

It is preferable that the composition of the present invention is administered to a subject under the mildly irritating condition. Administration under the mildly irritating condition can be attained, for example, by (i) administering the composition of the present invention to the subject under such an administration condition that transepidermal water loss (TEWL) (g/h·$m^2$) evaluated in a model animal for skin irritation evaluation is 50 or less, or (ii) administering to a subject the composition providing the cutaneous TSLP level (pg/mg protein) of 10000 or less evaluated in a model animal for evaluating skin irritation.

The therapeutically effective amount of the HER2/neu E75 peptide and/or the modified HER2/neu E75 peptide may widely vary depending on severity of the disease, age and relative health of the subject and other known factors. In general, satisfactory result may be obtained at a one day dose of about 0.1 μg to 1 g/kg body weight. The cellular immunity induction promoter is administered simultaneously with the HER2/neu E75 peptide and/or the modified HER2/neu E75 peptide or sequentially, and preferably, it is administered simultaneously with the peptide. The effective amount of the cellular immunity induction promoter may widely vary depending on the kind of cellular immunity induction promoter to be used, the presence or absence of other cellular immunity induction promoter and the like, and satisfactory result is obtained at a one day dose of about 0.01 μg to 1 g/kg body weight. The one day dose may be administered in a single dose or in several divided portions at several times such as two times or more, for example, two, three, four or

five times. The composition may be applied continuously for a period of between 1 minute and 7 days per one administration. The administration interval is appropriately selected from once every day to once per year (e.g. once per one day, once per 2 days, once per 3 days, once per one week, once per 2 weeks, once per one month, once per 3 months, once per 6 months, once per one year) and longer depending on the state of the patient, severity of the cancer, whether it is for therapeutic purpose or preventive purpose, or the like. Generally, for the purpose of treating a patient actually having a severe cancer, the HER2/neu E75 peptide and/or the modified HER2/neu E75 peptide are administered at a higher frequency and a higher dose, while for the preventive purpose for a patient having no cancer, the HER2/neu E75 peptide and/or the modified HER2/neu E75 peptide are administered at a lower frequency and a lower dose.

In the present invention, physical irritation means any physical irritation which gives damage to corneum, including scratch and scraping. For example, operation of tape stripping which removes corneum with an adhesive tape or the like, operation of giving damage to the skin with a cutter, and operation using a microneedle such as perforation in corneum are also included in the physical irritation.

Transepidermal water loss means the amount (g) of water which is transpired from 1 $m^2$ of keratin per one hour. The transepidermal water loss can be easily measured in a short time with a water loss measuring device, and is widely used as an index for evaluating the damage degree of the skin. Also in the present invention, the transepidermal water loss can be used as an index of the physical irritation level.

TSLP (Thymic stromal lymphopoietin) is one of IL-7-like cytokines which is produced by keratinocyte of skin, thymus, and mucosal epithelial cells, and is known to be involved in the maturation of dendritic cells, and the differentiation of T cells. In the present invention, the TSLP level can be used as an index of the chemical irritation level which is irritation derived from a drug.

The invention will be described in more detail and more specifically in the following Examples, but is not limited to the Examples.

EXAMPLES

Liquid Formulation for External Use

The liquid formulations for external use containing the ingredients as shown in Table 1 were prepared. Specifically, HER2/neu E75 peptide (TFA salt) and a cellular immunity induction promoter in the amounts as shown in Table 1, and 20 parts by weight of DMSO were blended to form a mixture, and a base material was added to the mixture so that the total amount is 100 parts by weight. Then blended the mixture was blended to form a liquid formulation for external use. The base material used was prepared by mixing and blending propylene glycol (PG) and oleyl alcohol (OA) at a weight ratio of 98 : 2 or 90 : 10. Imiquimod (IMQ) was purchased from Tokyo Chemical Industry Co., Ltd. The HER2/neu E75 peptide and the Peptide-25 was prepared by chemical synthesis, and purified by HPLC before use.

A cellulose non-woven fabric (area 0.8 $cm^2$) was attached to a middle part of an adhesive tape for fixing to prepare a composite substrate. The non-woven fabric part of the composite substrate was immersed with 67 $\mu$L of the liquid formulation for external use. Thus a sample for immunity induction test was prepared.

Immunity Induction Test 1 in Mouse (Liquid Formulation for External Use)

The liquid formulations for external use prepared as above described were used to perform an immunity induction test using a model mouse for immunological evaluation. The immunity induction level was evaluated by ELISPOT method. Specifically, the hair of the back skin of a mouse was cut, and the mouse was kept in a period for recovering from the skin damage. Thereafter, each sample was administered to the back skin of the mouse for a predetermined period, and then removed. After keeping the mouse for a predetermined day, the level of the cellular immunity induction specific for the antigen was evaluated. On a predetermined day after the administration, the spleen of the mouse was excised to prepare a suspension of the spleen cells. Spleen cells ($3\times10^6$ cells/well) and an antigen peptide (100 $\mu$M) were added together with a culture solution into a well of ELISPOT plate containing an immobilized anti-mouse IFN-$\gamma$ antigen, and co-cultured at 37°C in 5% $CO_2$ for 20 hours. The spot number of IFN-$\gamma$-producing cells was evaluated by ELISPOT method (the number of the spot/$3\times10^6$ cells). The dosage of the liquid formulation for external use was all 67 $\mu$L as described above, and the administration was performed once (24hr/week), the spleen was excised 6 days after the administration.

For some liquid formulations for external uses, cutaneous TSLP level in the skin of the mouse after administration and transepidermal water loss of the mouse before administration were also measured according to the method as described below. The mouse used for measuring TSLP level and transepidermal water loss is C57BL/6 mouse as a model animal for evaluating skin irritation.

(Method for Measuring TSLP Level)

When the application of the sample was completed, the skin of the back of the mouse was isolated, and the skin was ground using a homogenizer (Physcotron, Microtec Co., Ltd.) in an extraction solvent (PBS solution containing a protease inhibitor (Protease Inhibitor Cocktail for general use, manufactured by Sigma-Aldrich) and 10 μM indomethacin (manufactured by Wako Pure Chemical Industries, Ltd.)). The ground skin was centrifuged at 4°C and 9000 g for 10 minutes, and the supernatant was recovered. The TSLP amount in the supernatant was measured by ELISA (Mouse TSLP Quantikine ELISA Kit, manufactured by R&D Systems). In addition, the total protein amount in the supernatant was measured by the BCA method (Pierce BCA Protein Assay Kit, manufactured by Thermo Fisher Scientific K.K.), and the TSLP amount was divided by the total protein amount for standardization.

(Measurement of Transepidermal Water Loss)

A portable machine for measuring the water loss in closed chamber method (manufactured by AsahiBioMed Co., Ltd., VAPO SCAN AS-VT100RS) was used. The machine was contacted with the skin of mice for 5-15 seconds for measurement. At 10 minutes after the pretreatment of the skin of the mouse, the water loss was measured, and the value was considered as a transepidermal water loss (TEWL) ($g/h \cdot m^2$).

The results of the immunity induction test, and the measurement results of TSLP level and transepidermal water loss are shown in Table 1. The mouse used was a genetically modified mouse in which the cellular immunity induction by HLA-A* 0201 type MHC-restricted peptide can be evaluated. In addition, for the comparison, the result of the immunity test using injections as described below (Comparative Example 5) is shown in the last part of the Table.

Table 1

| | composition | | | | | chemical irritation | TSLP (pg/mg protein) | physical irritation | TEWL (g/h·$m^2$) | Results of immunization (ELISPOT average spot number) |
|---|---|---|---|---|---|---|---|---|---|---|
| | base | antigen peptide | cellular induction | immunity promoter | acid | | | | | |
| Comparative example 1 | PG/OA [98/2] | HER2/neu E75 (10) | none | none | none | none | | none | 10 | 5 |
| Example 1 | PG/OA [98/2] | HER2/neu E75 (10) | IMQ (3) | PEP (0.3) | none | none | 224 | none | 10 | 882 |
| Example 2 | PG/OA [98/2] | HER2/neu E75 (10) | IMQ (3) | PEP (0.3) | lactic acid (1) | none | | none | 10 | 1150 |
| Example 3 | PG/OA [98/2] | HER2/neu E75 (10) | IMQ (3) | PEP (0.3) | methanesulfonic acid (1) | none | | none | 10 | 967 |
| Example 4 | PG/OA [98/2] | HER2/neu E75 (10) | IMQ (3) | PEP (0.3) | D,L-malic acid (1) | none | | none | 10 | 933 |
| Example 5 | PG/OA [98/2] | HER2/neu E75 (10) | IMQ (3) | PEP (0.3) | maleic acid (1) | none | | none | 10 | 925 |
| Comparative example 5 | saline | HER2/neu E75 (0.125) | Montanide (50) | ISA51VG | none | none | | none | | 110 |

PG/OA: mixture of propylene glycol and oleyl alcohol (both are manufactured by Wako Pure Chemical Industries, Ltd.). The number in square brackets [] shows the ratio of PG to OA.
The number in parentheses () is the blending proportion (part(s) by weight) of each component. (It has the same meaning in the following Tables.)
IMQ: Imiquimod (TLR7 and/or TLR8 ligand)
PEP: Peptide-25 (SEQ ID NO: 3) (helper peptide)

Tape preparation

The adhesives (PIB rubber adhesive and acrylic adhesive) used in tape preparations were prepared as follows.

(Preparation of PIB Rubber Adhesive)

A PIB adhesive solution was obtained by dissolving 24 parts of a polyisobutylene (Oppanol B200, manufactured by BASF), 36 parts of a polyisobutylene (Oppanol B12, manufactured by BASF), and 40 parts of an alicyclic petroleum polymer resin (ARKON P-100, manufactured by ARAKAWA CHEMICAL INDUSTRIES, LTD.) in toluene.

(Polymerization of Acrylic Adhesive)

An acrylic adhesive solution was obtained by solution polymerization of 75 parts of 2-ethylhexyl acrylate, 22 parts of N-vinyl-2-pyrrolidone, 3 parts of acrylic acid and 0.2 part of azobisisobutyronitrile in ethyl acetate in inert gas atmosphere at 60°C.

The tape preparations containing the ingredients as shown in Table 2 were prepared. Specifically, the HER2/neu E75 peptide (TFA salt), a cellular immunity induction promoter in an amount as shown in Table 2, and optionally the skin permeability enhancer and/or a pharmacologically acceptable acid and/or an additive, the adhesive solution, and an organic solvent (for example, ethyl acetate, ethanol, or toluene) were mixed and blended to form a mixture. The mixture was spreaded on a release liner so that the thickness of the mixture after drying is about 80 μm. The mixture was then dried to remove any organic solvent, and attached to a support to form a tape preparation. The adhesive solution was blended so that total amount of each components and the adhesive is 100 parts by weight after drying the organic solvent. The tape preparation was cut into a piece having an area of 0.7 $cm^2$. The piece was used as a sample for immunity induction test. When administered, the release liner was removed off and the tape preparation was administered.

Apolyethylene terephthalate (PET) film (thickness 25 μm) was used as the support. A release liner made by polyethylene terephthalate (PET) (thickness 75 μm) after silicone releasing process was used as the release liner. The cyclic diGMP (c-di-GMP) and the cyclic diAMP (c-di-AMP) were purchased from Biolog Life Science Institute. The lipopolysaccharide derived from Pantoea was manufactured by Institute of applied technology for innate immunity. Glucopyranosyl lipid was manufactured by InvivoGen (MPLAs). Hyaluronic acid Na was manufactured by Kikkoman Biochemifa Company (microhyaluronic acid FCH). ODN1826 was manufactured by InvivoGen. Levamisole hydrochloride was manufactured by MP Biomedical. Pidotimod was manufactured by Santa Cruz Biotechnology, Inc. Etodolac was manufactured by Wako Pure Chemical Industries, Ltd. Loxoprofen Na was manufactured by Yoshindo Inc. Quercetin was manufactured by Cayman Chemical Company. PADRE, HER2/neu E75 peptide, and Peptide-25 were chemically synthesized, and purified by HPLC before use. Imiquimod was obtained from the same supplier as described above for liquid formulations for external use.

Immunity Induction Test 2 in Mouse (Tape preparation)

The tape preparations prepared as described above were used to perform an immunity induction test according to the same method as Immunity Induction Test 1. The administration was performed once (24hr/week), and the spleen was excised 6 days after the administration.

In Example 9, the formulations were administered to the skin damaged by a micro cutter (MICRO FEATHER No.7330G, manufactured by FEATHER).

For some tape preparations, cutaneous TSLP level in the skin of the mouse after administration and transepidermal water loss of the mouse before administration were also measured according to the method described above for liquid formulations for external use. The mouse used for measuring TSLP level and transepidermal water loss was C57BL/6 mouse.

The results of the immunity induction test, and the measurement results of TSLP level and transepidermal water loss are shown in Table 2. The mouse used was a genetically modified mouse in which the cellular immunity induction by HLA-A* 0201 type MHC-restricted peptide can be evaluated. In addition, for the comparison, the result of the immunity test using injections as described below (Comparative Example 5) is shown in the last part of the Table.

Table 2

| | Composition | | | | | | | TSLP (pg/mg protein) | physical irritation | TEWL ($g/hm^2$) | Results of immunization (ELISPOT average spot number) | % Specific Lysis (In vivo CTL assay) |
|---|---|---|---|---|---|---|---|---|---|---|---|---|
| | base | antigen peptide | cellular immunity induction promoter | | skin permeability enhancer | acid | additive (chemical irritation) | | | | | |
| Comparative example 2 | acryl | HER2/neu E75 (10) | None | None | IPM (34.4) | None | None | 32 | None | 12 | 5 | 2 |
| Example 6 | acryl | HER2/neu E75 (10) | IMQ(3) | None | IPM (34.4) | None | None | 50 | None | 12 | 55 | |
| Example 7 | acryl | HER2/neu E75 (10) | None | PEP (1) | IPM (34.4) | None | None | 35 | None | 12 | 14 | |
| Example 8 | acryl | HER2/neu E75 (10) | IMQ(3) | PEP (1) | IPM (34.4) | None | containing 10% SDS | 550 | None | 12 | 58 | |
| Example 9 | acryl | HER2/neu E75 (10) | IMQ(3) | PEP (1) | IPM (34.4) | None | None | 48 | microcutter | 64 | 55 | |
| Example 10 | acryl | HER2/neu E75 (10) | IMQ(3) | PEP (1) | IPM (34.4) | None | None | 52 | None | 10 | 78 | |
| Example 11 | acryl | HER2/neu E75 (10) | IMQ(3) | PEP (1) | IPM (25.8) | myristic acid (8.6) | None | 98 | None | 10 | 117 | 30 |
| Example 12 | PIB | HER2/neu E75 (10) | IMQ(3) | PEP (1) | IPM (34.4) | None | None | 25 | None | 10 | 23 | |
| Example 13 | acryl | HER2/neu E75 (10) | c-di-GMP (cyclic dinucleotide) (1) | PEP (1) | IPM (34.4) | None | None | | None | | | |
| Example 14 | acryl | HER2/neu E75 (10) | c-di-AMP(cyclic dinucleotide) (1) | PEP (1) | IPM (34.4) | None | None | | None | | | |
| Example 15 | acryl | HER2/neu E75 (10) | lipopolysaccharide derived from Pantoea bacterium (TLR4 ligand) (1) | None | IPM (34.4) | None | None | 36 | None | 12 | 33 | |

(continued)

| | Composition | | | | | | | TSLP (pg/mg protein) | physical irritation | TEWL (g/hm$^2$) | Results of immunization (ELISPOT average spot number) | % Specific Lysis (In vivo CTL assay) |
|---|---|---|---|---|---|---|---|---|---|---|---|---|
| | base | antigen peptide | cellular immunity induction promoter | | skin permeability enhancer | acid | additive (chemical irritation) | | | | | |
| Example 16 | acryl | HER2/neu E75 (10) | lipopolysaccharide derived from Pantoea bacterium (TLR4 ligand) (1) | PEP (1) | IPM (34.4) | None | None | 40 | None | 12 | 52 | |
| Example 17 | acryl | HER2/neu E75 (10) | glucopyranosyl lipid (TLR4 ligand) (1) | PEP (1) | IPM (34.4) | None | None | | None | | | |
| Example 18 | acryl | HER2/neu E75 (10) | sodium hyaluronate (TLR4 ligand) (1) | PEP (1) | IPM (34.4) | None | None | | None | | | |
| Example 19 | acryl | HER2/neu E75 (10) | ODN1826 (TLR9 ligand) (1) | PEP (1) | IPM (34.4) | None | None | | None | | | |
| Example 20 | acryl | HER2/neu E75 (10) | levamisole hydrochloride (immunomodulatory small molecule drug) (1) | None | IPM (34.4) | None | None | 33 | None | 12 | 15 | |
| Example 21 | acryl | HER2/neu E75 (10) | levamisole hydrochloride (immunomodulatory small molecule drug) (1) | PEP (1) | IPM (34.4) | None | None | 39 | None | 12 | 32 | |
| Example 22 | acryl | HER2/neu E75 (10) | pidotimod (immunomodulatory small molecule drug) (1) | None | IPM (34.4) | None | None | 31 | None | 12 | 14 | |
| Example 23 | acryl | HER2/neu E75 (10) | pidotimod (immunomodulatory small molecule drug) (1) | PEP (1) | IPM (34.4) | None | None | 35 | None | 12 | 30 | |
| Example 24 | acryl | HER2/neu E75 (10) | etodolac(COX inhibitor) (1) | None | IPM (34.4) | None | None | 28 | None | 12 | 29 | |

(continued)

| | Composition | | | | | | | TSLP (pg/mg protein) | physical irritation | TEWL (g/hm$^2$) | Results of immunization (ELISPOT average spot number) | % Specific Lysis (In vivo CTL assay) |
|---|---|---|---|---|---|---|---|---|---|---|---|---|
| | base | antigen peptide | cellular immunity induction promoter | | skin permeability enhancer | acid | additive (chemical irritation) | | | | | |
| Example 25 | acryl | HER2/neu E75 (10) | etodolac (COX inhibitor) (1) | PEP (1) | IPM (34.4) | None | None | 30 | None | 12 | 87 | |
| Example 26 | acryl | HER2/neu E75 (10) | loxoprofen Na(COX inhibitor) (3) | None | IPM (34.4) | None | None | 27 | None | 12 | 21 | |
| Example 27 | acryl | HER2/neu E75 (10) | loxoprofen Na(COX inhibitor) (3) | PEP (1) | IPM (34.4) | None | None | 30 | None | 12 | 64 | |
| Example 28 | acryl | HER2/neu E75 (10) | loxoprofen Na(COX inhibitor) (1.5)+ IMQ (1.5) | PEP (1) | IPM (34.4) | None | None | 36 | None | 12 | 101 | 28 |
| Example 29 | acryl | HER2/neu E75 (10) | quercetin (TSLP production inhibitor) (3) | None | IPM (34.4) | None | None | 27 | None | 12 | 18 | |
| Example 30 | acryl | HER2/neu E75 (10) | quercetin(TSLP production inhibitor) (3) | PEP (1) | IPM (34.4) | None | None | 28 | None | 12 | 47 | |
| Example 31 | acryl | HER2/neu E75 (10) | quercetin (TSLP production inhibitor) (1.5)+ IMQ (1.5) | PEP (1) | IPM (34.4) | None | None | 33 | None | 12 | 127 | 32 |
| Example 32 | acryl | HER2/neu E75 (10) | IMQ (3) | PADRE (1) | IPM (34.4) | None | None | | None | 12 | 68 | |
| Example 33 | acryl | HER2/neu E75 (10) | IMQ (3) | PADRE (1) | IPM (14.4) | lactic acid (20.0) | None | | None | 12 | 210 | |
| Example 34 | acryl | HER2/neu E75 (10) | IMQ (3) | PEP (1) | IPP (34.4) | None | None | | None | 12 | 65 | |
| Example 35 | acryl | HER2/neu E75 (10) | IMQ (3) | PEP (1) | None | None | None | | None | 12 | 35 | |

(continued)

| | Composition | | | | | | TSLP (pg/mg protein) | physical irritation | TEWL ($g/hm^2$) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | skin permeability enhancer | acid | additive (chemical irritation) | | | | | |
| Comparative example 5 | saline | HER2/neu E75 (0.125) | Montanide ISA51VG (50) | None | None | None | | None | | 110 | 28 |

acryl: acrylic adhesive
PIB:PIB rubber adhesive
c-di-GMP: cyclic diGMP
c-di-AMP: cyclic diAMP
PADRE: universal helper peptide (SEQ ID NO: 4) (helper peptide)
IPM: isopropyl myristate, manufactured by Croda Japan KK
IPP: isopropyl palmitate, manufactured by Wako Pure Chemical Industries, Ltd.
MA: myristic acid
SDS: sodium dodecyl sulfate

Cream Formulation

Cream formulation containing the ingredients as shown in Table 4 were prepared. Specifically, HER2/neu E75 peptide (TFA salt) and a cellular immunity induction promoter in the amounts as shown in Table 4, 15 parts by weight of DMSO, and optionally an additive were blended to form a mixture, and added a base (a base cream or an acid-free cream) to the mixture so that the total amount is 100 parts by weight, and then blended the mixture to form a cream formulation. The base cream and the acid-free cream used were prepared by mixing and blending the materials as shown in Table 3-1 and Table 3-2, respectively.

A composite substrate in which a PET film/PET non-woven fabric laminate product (area 0.7 $cm^2$) was stuck to the central portion of an adhesive tape for fixation so that the PET film side faces the tape was prepared. The cream formulation (4 mg) was coated on the non-woven fabric portion of this composite substrate, and this was used in the immunization test.

HER2/neu E75 peptide and the cellular immunity induction promoter were obtained from the same supplier as described above for liquid formulations for external use and tape preparations.

Immunity Induction Test 3 in Mouse (Cream Formulation)

The cream formulations prepared as described above were used to perform an immunity induction test according to the same method as Immunity Induction Test 1. The number of the administration was one (24hr/week), and the spleen was excised 6 days after the administration.

In Example 36, the formulations were administered to the skin damaged by a micro cutter (MICRO FEATHER No.7330G, manufactured by FEATHER).

For some cream formulations, according to the method described for formulations for external use as described below, cutaneous TSLP level in the skin of the mouse after administration and transepidermal water loss of the mouse before administration were also measured. The mouse used for measuring TSLP level and transepidermal water loss is C57BL/6 mouse as a model animal for evaluating skin irritation.

The results of the immunity induction test, and the measurement results of TSLP level and transepidermal water loss are shown in Table 4. The mouse used was a genetically modified mouse in which the cellular immunity induction by HLA-A* 0201 type MHC-restricted peptide can be evaluated. In addition, for the comparison, the result of the immunity test using injections as described below (Comparative Example 5) is shown in the last part of the Table.

Table 3-1

| | base cream |
|---|---|
| white vaseline | 60.7 wt% |
| Sorbitan monostearate | 0.7 wt% |
| isostearic acid | 12.0 wt% |
| benzyl alcohol | 2.4 wt% |
| cetanol | 2.4 wt% |
| stearyl alcohol | 3.5 wt% |
| polysorbate 60 | 3.5 wt% |
| concentrated glycerin | 2.4 wt% |
| water | 12.4 wt% |

Table 3-2

| | acid-free cream |
|---|---|
| white vaseline | 69.0 wt% |
| Sorbitan monostearate | 0.8 wt% |
| benzyl alcohol | 2.7 wt% |
| cetanol | 2.7 wt% |

(continued)

| | acid-free cream |
|---|---|
| stearyl alcohol | 4.0 wt% |
| polysorbate 60 | 4.0 wt% |
| concentrated glycerin | 2.7 wt% |
| water | 14.1 wt% |

White vaseline, sorbitan monostearate, isostearic acid, benzyl alcohol, stearyl alcohol, polysorbate 60, and concentrated glycerin were purchased from Wako Pure Chemical Industries, Ltd. Cetanol was purchased from Tokyo Chemical Industry Co., Ltd.

The following materials were used: imiquimod: manufactured by Tokyo Chemical Industry Co., Ltd., R848: manufactured by InvivoGen, clofibrate: manufactured by LKT Laboratories, fenofibrate: manufactured by Wako Pure Chemical Industries, Ltd., quercetin: manufactured by Cayman Chemical, lipopolysaccharide derived from Pantoea bacterium: manufactured by MACROPHI Inc., glucopyranosyl lipid: manufactured by InvivoGen (MPLAs), sodium hyaluronate manufactured by Kikkoman Biochemifa Company (microhyaluronic acid FCH), berberine (berberine chloride n hydrates): manufactured by Wako Pure Chemical Industries, Ltd., 3,3'-diindolylmethane: manufactured by Wako Pure Chemical Industries, Ltd., xanthone: manufactured by Wako Pure Chemical Industries, Ltd., parthenolide: manufactured by Wako Pure Chemical Industries, Ltd., noscapine: manufactured by Wako Pure Chemical Industries, Ltd., etodolac: manufactured by Wako Pure Chemical Industries, Ltd., loxoprofen (loxoprofen Na): manufactured by Yoshindo Inc., indomethacin: manufactured by Wako Pure Chemical Industries, Ltd., aspirin: manufactured by Sigma-Aldrich, diclofenac (diclofenac sodium): manufactured by Wako Pure Chemical Industries, Ltd., ketoprofen: manufactured by Wako Pure Chemical Industries, Ltd., celecoxib: manufactured by TOCRIS bioscience, valdecoxib: manufactured by TOCRIS bioscience, GW627368X: manufactured by Cayman Chemical Company, BWA868C: manufactured by Cayman Chemical Company, RO1138452: manufactured by Cayman Chemical Company, sulprostone: manufactured by Cayman Chemical Company, cloprostenol: manufactured by Wako Pure Chemical Industries, Ltd., docosahexaenoic acid: manufactured by Cayman Chemical, 2',5'-dideoxyadenosine: BIOMOL International, SCH23390: manufactured by Wako Pure Chemical Industries, Ltd., ropinirole (ropinirole hydrochloride): manufactured by Ragactives, leukotriene B4: manufactured by Cayman Chemical, montelukast (montelukast sodium): LG Life Sciences, glycyrrhizic acid (glycyrrhizinate dipotassium): manufactured by Wako Pure Chemical Industries, Ltd., pirfenidone: manufactured by TOCRIS bioscience, tranilast: manufactured by Wako Pure Chemical Industries, Ltd., famotidine: manufactured by Wako Pure Chemical Industries, Ltd., Impip (Impip dihydrobromate): manufactured by TOCRIS bioscience, proxyfan: manufactured by TOCRIS bioscience, azelastine (azelastine hydrochloride): LKT Labs, cimetidine: manufactured by Wako Pure Chemical Industries, Ltd., 4-methylhistamine: manufactured by TOCRIS bioscience, olanzapine: manufactured by Wako Pure Chemical Industries, Ltd., yohimbine (yohimbine hydrochloride): manufactured by Wako Pure Chemical Industries, Ltd., acetylcholine (acetylcholine chloride) : manufactured by Wako Pure Chemical Industries, Ltd., metergoline (metergoline phenylmethyl ester): manufactured by TOCRIS bioscience, clozapine: manufactured by Wako Pure Chemical Industries, Ltd., sumatriptan: manufactured by MYUNG IN PHARM, zolmitriptan: manufactured by Cipla, tolvaptan: manufactured by Sigma-Aldrich, desmopressin: manufactured by Sigma-Aldrich, oxybutynin (oxybutynin hydrochloride): manufactured by Sigma-Aldrich, tamsulosin (tamsulosin hydrochloride): manufactured by Cipla, pilocarpine (pilocarpine hydrochloride): manufactured by Wako Pure Chemical Industries, Ltd., propranolol (propranolol hydrochloride): manufactured by Wako Pure Chemical Industries, Ltd., xylazine: manufactured by Wako Pure Chemical Industries, Ltd., novokinin: manufactured by Sigma-Aldrich, baclofen: manufactured by Tokyo Chemical Industry Co., Ltd., TRAP-6: manufactured by Bachem, adenosine diphosphate: manufactured by MP Biomedicals, somatostatin-14: manufactured by Bachem, GW405833: manufactured by Sigma-Aldrich, SEW2871: manufactured by Cayman Chemical, trimebutine (trimebutine maleate): manufactured by Tokyo Chemical Industry Co., Ltd., loperamide (loperamide hydrochloride): manufactured by Wako Pure Chemical Industries, Ltd., melatonin: LKT Labs, biphenylindanone A: manufactured by Sigma-Aldrich, L-AP4 (L-2-amino-4-phosphonobutyric acid): manufactured by Wako Pure Chemical Industries, Ltd., diphenhydramine (diphenhydramine hydrochloride): manufactured by Wako Pure Chemical Industries, Ltd., cimetidine: manufactured by Wako Pure Chemical Industries, Ltd., and suplatast tosylate manufactured by TOCRIS bioscience.

Table 4

| | composition | | | | | | TSLP (pg/mg protein) | physical irritation | TEWL (g/h-m$^2$) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | | acid | additive (chemical irritation) | | | | | |
| Example 36 | base cream | HER2/neu E75 (25) | IMQ (3) | PEP (0.3) | ISA (in base cream) | None | 67 | microcutter | 66 | 73 | |
| Example 37 | base cream | HER2/neu E75 (25) | IMQ (3) | PEP (0.3) | ISA (in base cream) | None | 78 | None | 10 | 123 | 30 |
| Comparative example 3 | base cream | HER2/neu E75 (10) | None | None | ISA (in base cream) | None | 70 | None | 12 | 6 | |
| Example 38 | base cream | HER2/neu E75 (10) | None | PEP (1) | ISA (in base cream) | None | 74 | None | 12 | 20 | |
| Example 39 | base cream | HER2/neu E75 (10) | R848 (TLR7 and/or TLR8 ligand) (3) | PEP (1) | ISA (in base cream) | None | | None | 12 | 138 | |
| Example 40 | base cream | HER2/neu E75 (10) | lipopolysaccharide derived from Pantoea bacterium (TLR4 ligand) (3) | PEP (1) | ISA (in base cream) | None | | None | 12 | 102 | |
| Example 41 | base cream | HER2/neu E75 (10) | glucopyranosyl lipid (TLR4 ligand) | PEP (1) | ISA (in base cream) | None | | None | 12 | 95 | |
| Example 42 | base cream | HER2/neu E75 (10) | sodium hyaluronate (TLR4 ligand) (3) | PEP (1) | ISA (in base cream) | None | | None | 12 | 88 | |
| Example 43 | base cream | HER2/neu E75 (10) | c-di-GMP (0.3) | PEP (1) | ISA (in base cream) | None | | None | 12 | 155 | |
| Example 44 | base cream | HER2/neu E75 (10) | c-di-AMP (0.3) | PEP (1) | ISA (in base cream) | None | | None | 12 | 143 | |
| Example 45 | base cream | HER2/neu E75 (10) | pidotimod(immunomodulatory small molecule drug) (3) | PEP (1) | ISA (in base cream) | None | | None | 12 | 83 | |

(continued)

| | composition | | | | | | TSLP (pg/mg protein) | physical irritation | TEWL (g/h-m$^2$) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | | acid | additive (chemical irritation) | | | | | |
| Example 46 | base cream | HER2/neu E75 (10) | Bestatin(immunomodulatory small molecule drug) (3) | PEP (1) | ISA (in base cream) | None | | None | 12 | 75 | |
| Example 47 | base cream | HER2/neu E75 (10) | levamisole hydrochloride (immun omodulatory small molecule drug) (3) | PEP (1) | ISA (in base cream) | None | | None | 12 | 112 | |
| Example 48 | base cream | HER2/neu E75 (10) | loxoprofen Na(COX inhibitor) (3) | PEP (1) | ISA (in base cream) | None | 58 | None | 12 | 114 | |
| Example 49 | base cream | HER2/neu E75 (10) | etodolac(COX inhibitor) (3) | PEP (1) | ISA (in base cream) | None | | None | 12 | 98 | |
| Example 50 | base cream | HER2/neu E75 (10) | diclofenac(COX inhibitor) (3) | PEP (1) | ISA (in base cream) | None | | None | 12 | 132 | |
| Example 51 | base cream | HER2/neu E75 (10) | indomethacin(COX inhibitor) (3) | PEP (1) | ISA (in base cream) | None | | None | 12 | 123 | |
| Example 52 | base cream | HER2/neu E75 (10) | aspirin(COX inhibitor) (3) | PEP (1) | ISA (in base cream) | None | | None | 12 | 131 | |
| Example 53 | base cream | HER2/neu E75 (10) | ketoprofen(COX inhibitor) (3) | PEP (1) | ISA (in base cream) | None | | None | 12 | 138 | |
| Example 54 | base cream | HER2/neu E75 (10) | celecoxib (COX inhibitor) (3) | PEP (1) | ISA (in base cream) | None | | None | 12 | 121 | |
| Example 55 | base cream | HER2/neu E75 (10) | valdecoxib (COX inhibitor) (3) | PEP (1) | ISA (in base cream) | None | | None | 12 | 115 | |
| Example 56 | base cream | HER2/neu E75 (10) | quercetin (TSLP production inhibitor) (3) | None | ISA (in base cream) | None | 50 | None | 12 | 75 | |
| Example 57 | base cream | HER2/neu E75 (10) | quercetin(TSLP production inhibitor) (3) | PEP (1) | ISA (in base cream) | None | 52 | None | 12 | 108 | |

(continued)

| | composition | | | | | | TSLP (pg/mg protein) | physical irritation | TEWL (g/h-m$^2$) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | | acid | additive (chemical irritation) | | | | | |
| Example 58 | base cream | HER2/neu E75 (10) | berberine (TSLP production inhibitor) (3) | PEP (1) | ISA (in base cream) | None | | None | 12 | 112 | |
| Example 59 | base cream | HER2/neu E75 (10) | 3,3'-diindolylmetha ne (TSLP production inhibitor) (3) | PEP (1) | ISA (in base cream) | None | | None | 12 | 114 | |
| Example 60 | base cream | HER2/neu E75 (10) | xanthone (TSLP production inhibitor) (3) | PEP (1) | ISA (in base cream) | None | | None | 12 | 120 | |
| Example 61 | base cream | HER2/neu E75 (10) | parthenolide (TSLP production inhibitor) (3) | PEP (1) | ISA (in base cream) | None | | None | | | |
| Example 62 | base cream | HER2/neu E75 (10) | noscapine (TSLP production inhibitor) (3) | PEP (1) | ISA (in base cream) | None | | None | | | |
| Example 63 | base cream | HER2/neu E75 (10) | GW627368X (prostagla ndin receptor antagonist) (3) | PEP (1) | ISA (in base cream) | None | | None | 12 | 141 | |
| Example 64 | base cream | HER2/neu E75 (10) | BWA868C (prostagland in receptor antagonist) (3) | PEP (1) | ISA (in base cream) | None | | None | 12 | 134 | |
| Example 65 | base cream | HER2/neu E75 (10) | RO1138452 (prostagla ndin receptor antagonist) (3) | PEP (1) | ISA (in base cream) | None | | None | 12 | 122 | |
| Example 66 | base cream | HER2/neu E75 (10) | cloprostenol (prostaglandin receptor agonist) (3) | PEP (1) | ISA (in base cream) | None | | None | 12 | 115 | |
| Example 67 | base cream | HER2/neu E75 (10) | sulprostone (prostag landin receptor agonist) (3) | PEP (1) | ISA (in base cream) | None | | None | 12 | 118 | |
| Example 68 | base cream | HER2/neu E75 (10) | docosahexaenoic acid (omega-3 fatty acid) (3) | PEP (1) | ISA (in base cream) | None | | None | 12 | 43 | |

(continued)

| | composition | | | | | | TSLP (pg/mg protein) | physical irritation | TEWL (g/h-m2) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | | acid | additive (chemical irritation) | | | | | |
| Example 69 | base cream | HER2/neu E75 (10) | 2',5'-dideoxyadenos ine (adenylate cyclase inhibitor) (3) | PEP (1) | ISA (in base cream) | None | | None | 12 | 54 | |
| Example 70 | base cream | HER2/neu E75 (10) | clofibrate (PPAR agonist) (3) | PEP (1) | ISA (in base cream) | None | | None | 12 | 102 | |
| Example 71 | base cream | HER2/neu E75 (10) | fenofibrate (PPAR agonist) (3) | PEP (1) | ISA (in base cream) | None | | None | 12 | 89 | |
| Example 72 | base cream | HER2/neu E75 (10) | SCH23390 (dopamine receptor antagonist) (3) | PEP (1) | ISA (in base cream) | None | | None | 12 | 61 | |
| Example 73 | base cream | HER2/neu E75 (10) | ropinirole (dopamine receptor agonist) (3) | PEP (1) | ISA (in base cream) | None | | None | 12 | 54 | |
| Example 74 | base cream | HER2/neu E75 (10) | Immepip (histamine receptor agonist) (3) | PEP (1) | ISA (in base cream) | None | | None | | | |
| Example 75 | base cream | HER2/neu E75 (10) | proxyfan (histamine receptor agonist) (3) | PEP (1) | ISA (in base cream) | None | | None | 12 | 85 | |
| Example 76 | base cream | HER2/neu E75 (10) | 4-methylhistamine (h istamine receptor agonist) (3) | PEP (1) | ISA (in base cream) | None | | None | 12 | 83 | |
| Example 77 | base cream | HER2/neu E75 (10) | azelastine (histamin e receptor antagonist) (3) | PEP (1) | ISA (in base cream) | None | | None | 12 | 121 | |
| Example 78 | base cream | HER2/neu E75 (10) | diphenhydramine (his tamine receptor antagonist) (3) | PEP (1) | ISA (in base cream) | None | | None | 12 | 112 | |
| Example 79 | base cream | HER2/neu E75 (10) | cimetidine (histamin e receptor antagonist) (3) | PEP (1) | ISA (in base cream) | None | | None | 12 | 118 | |
| Example 80 | base cream | HER2/neu E75 (10) | famotidine (histamin e receptor antagonist) (3) | PEP (1) | ISA (in base cream) | None | | None | 12 | 131 | |

(continued)

| | composition | | | | | | TSLP (pg/mg protein) | physical irritation | TEWL (g/h-m²) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | | acid | additive (chemical irritation) | | | | | |
| Example 81 | base cream | HER2/neu E75 (10) | sumatriptan (seroton in receptor agonist) (3) | PEP (1) | ISA (in base cream) | None | | None | 12 | 109 | |
| Example 82 | base cream | HER2/neu E75 (10) | zolmitriptan (seroto nin receptor agonist) (3) | PEP (1) | ISA (in base cream) | None | | None | 12 | 119 | |
| Example 83 | base cream | HER2/neu E75 (10) | yohimbine (serotonin receptor antagonist) (3) | PEP (1) | ISA (in base cream) | None | | None | | | |
| Example 84 | base cream | HER2/neu E75 (10) | olanzapine (serotoni n receptor antagonist) (3) | PEP (1) | ISA (in base cream) | None | | None | 12 | 82 | |
| Example 85 | base cream | HER2/neu E75 (10) | metergoline (seroton in receptor antagonist) (3) | PEP (1) | ISA (in base cream) | None | | None | 12 | 75 | |
| Example 86 | base cream | HER2/neu E75 (10) | clozapine (serotonin receptor antagonist) (3) | PEP (1) | ISA (in base cream) | None | | None | | | |
| Example 87 | base cream | HER2/neu E75 (10) | tolvaptan (vasopress in receptor antagonist) (3) | PEP (1) | ISA (in base cream) | None | | None | 12 | 61 | |
| Example 88 | base cream | HER2/neu E75 (10) | desmopressin (vasopressin receptor agonist) (3) | PEP (1) | ISA (in base cream) | None | | None | 12 | 53 | |
| Example 89 | base cream | HER2/neu E75 (10) | oxybutynin (muscarin e receptor antagonist) (3) | PEP (1) | ISA (in base cream) | None | | None | | | |
| Example 90 | base cream | HER2/neu E75 (10) | acetylcholine (musca rine receptor antagonist) (3) | PEP (1) | ISA (in base cream) | None | | None | 12 | 78 | |
| Example 91 | base cream | HER2/neu E75 (10) | trimebutine (muscari ne receptor antagonist) (3) | PEP (1) | ISA (in base cream) | None | | None | 12 | 72 | |
| Example 92 | base cream | HER2/neu E75 (10) | pilocarpine (muscari ne receptor agonist) (3) | PEP (1) | ISA (in base cream) | None | | None | 12 | 49 | |
| Example 93 | base cream | HER2/neu E75 (10) | tamsulosin (adrenali n receptor antagonist) (3) | PEP (1) | ISA (in base cream) | None | | None | | | |

(continued)

| | composition | | | | | | TSLP (pg/mg protein) | physical irritation | TEWL (g/h-m$^2$) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | | acid | additive (chemical irritation) | | | | | |
| Example 94 | base cream | HER2/neu E75 (10) | propranolol (adrenalin receptor antagonist) (3) | PEP (1) | ISA (in base cream) | None | | None | 12 | 83 | |
| Example 95 | base cream | HER2/neu E75 (10) | xylazine (adrenalin receptor agonist) (3) | PEP (1) | ISA (in base cream) | None | | None | 12 | 51 | |
| Example 96 | base cream | HER2/neu E75 (10) | novokinin (angiotens in receptor agonist) (3) | PEP (1) | ISA (in base cream) | None | | None | 12 | 61 | |
| Example 97 | base cream | HER2/neu E75 (10) | baclofen (GABA receptor agonist) (3) | PEP (1) | ISA (in base cream) | None | | None | 12 | 58 | |
| Example 98 | base cream | HER2/neu E75 (10) | TRAP-6 (thrombin receptor agonist) (3) | PEP (1) | ISA (in base cream) | None | | None | 12 | 65 | |
| Example 99 | base cream | HER2/neu E75 (10) | loperamide (opioid receptor agonist) (3) | PEP (1) | ISA (in base cream) | None | | None | 12 | 121 | |
| Example 100 | base cream | HER2/neu E75 (10) | adenosine diphosphate (ADP receptor agonist) (3) | PEP (1) | ISA (in base cream) | None | | None | 12 | 43 | |
| Example 101 | base cream | HER2/neu E75 (10) | montelukast (leukotr iene receptor antagonist) (3) | PEP (1) | ISA (in base cream) | None | | None | 12 | 52 | |
| Example 102 | base cream | HER2/neu E75 (10) | leukotriene B4 (leukotriene receptor agonist) (3) | PEP (1) | ISA (in base cream) | None | | None | 12 | 60 | |
| Example 103 | base cream | HER2/neu E75 (10) | melatonin (melatonin receptor agonist) (3) | PEP (1) | ISA (in base cream) | None | | None | 12 | 113 | |
| Example 104 | base cream | HER2/neu E75 (10) | somatostatin-14 (som atostatin receptor agonist) (3) | PEP (1) | ISA (in base cream) | None | | None | 12 | 58 | |
| Example 105 | base cream | HER2/neu E75 (10) | GW405833 (cannabinoid receptor agonist) (3) | PEP (1) | ISA (in base cream) | None | | None | 12 | 63 | |

(continued)

| | composition | | | | | | TSLP (pg/mg protein) | physical irritation | TEWL (g/h-$m^2$) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | | acid | additive (chemical irritation) | | | | | |
| Example 106 | base cream | HER2/neu E75 (10) | SEW2871 (sphingosine -1 phosphate receptor agonist) (3) | PEP (1) | ISA (in base cream) | None | | None | 12 | 48 | |
| Example 107 | base cream | HER2/neu E75 (10) | L-AP4 (metabotropic glutamate receptor agonist) (3) | PEP (1) | ISA (in base cream) | None | | None | 12 | 85 | |
| Example 108 | base cream | HER2/neu E75 (10) | biphenylindanone A (metabotropic glutamate receptor agonist) (3) | PEP (1) | ISA (in base cream) | None | | None | 12 | 80 | |
| Examples 109 | base cream | HER2/neu E75 (10) | dipotassium glycyrrhizinate (pho spholipase A2 inhibitor) (3) | PEP (1) | ISA (in base cream) | None | | None | 12 | 41 | |
| Example 110 | base cream | HER2/neu E75 (10) | tranilast (TGF-beta production inhibitor) (3) | PEP (1) | ISA (in base cream) | None | | None | 12 | 82 | |
| Example 111 | base cream | HER2/neu E75 (10) | pirfenidone (TGF-bet a production inhibitor) (3) | PEP (1) | ISA (in base cream) | None | | None | 12 | 72 | |
| Example 112 | base cream | HER2/neu E75 (10) | suplatast tosylate (Th2 cytokine inhibitor) (3) | PEP (1) | ISA (in base cream) | None | | None | 12 | 51 | |
| Comparative example 4 | acid-free cream | HER2/neu E75 (5) | None | None | None | None | | None | 12 | 3 | |
| Example 113 | acid-free cream | HER2/neu E75 (5) | IMQ (3) | None | None | None | | None | 12 | 34 | |
| Example 114 | acid-free cream | HER2/neu E75 (5) | IMQ (3) | PADRE (1) | None | None | | None | 12 | 45 | |
| Example 115 | acid-free cream | HER2/neu E75 (5) | IMQ (3) | PADRE (1) | myristic acid (5) | None | | None | 12 | 106 | |

(continued)

| | composition | | | | | | TSLP (pg/mg protein) | physical irritation | TEWL (g/h-m$^2$) | Results of immunization (ELISPOT average spot number) | % Specific Lysis (In vivo CTL assay) |
|---|---|---|---|---|---|---|---|---|---|---|---|
| | base | antigen peptide | cellular immunity induction promoter | | acid | additive (chemical irritation) | | | | | |
| Example 116 | acid-free cream | HER2/neu E75 (5) | IMQ (3) | PADRE (1) | ISA (5) | None | | None | 12 | 65 | |
| Example 117 | acid-free cream | HER2/neu E75 (5) | IMQ (3) | PADRE (1) | lauric acid (5) | None | | None | 12 | 110 | |
| Example 118 | acid-free cream | HER2/neu E75 (5) | IMQ (3) | PADRE (1) | decanoic acid (5) | None | | None | 12 | 123 | |
| Example 119 | acid-free cream | HER2/neu E75 (5) | IMQ (3) | PADRE (1) | oleic acid (5) | None | | None | 12 | 97 | |
| Example 120 | acid-free cream | HER2/neu E75 (5) | IMQ (3) | PADRE (1) | palmitic acid (5) | None | | None | 12 | 143 | |
| Example 121 | acid-free cream | HER2/neu E75 (5) | IMQ (3) | PADRE (1) | stearic acid (5) | None | | None | 12 | 140 | |
| Example 122 | acid-free cream | HER2/neu E75 (5) | IMQ (3) | PADRE (1) | lactic acid (5) | None | | None | 12 | 150 | |
| Example 123 | acid-free cream | HER2/neu E75 (5) | IMQ (3) | PADRE (1) | methanesulfonic acid (5) | None | | None | 12 | 138 | |
| Example 124 | acid-free cream | HER2/neu E75 (5) | IMQ (3) | PADRE (1) | malic acid (5) | None | | None | 12 | 82 | |
| Example 125 | acid-free cream | HER2/neu E75 (5) | IMQ (3) | PADRE (1) | salicylic acid (5) | None | | None | 12 | 132 | |
| Comparative example 5 | saline | HER2/neu E75 (0.125) | Montanide ISA51VG (50) | | | None | | None | | 110 | 28 |
| ISA: isostearic acid | | | | | | | | | | | |

Subcutaneous Injectable Formulations

The subcutaneous injectable formulations containing the ingredients as shown in Table 5 were prepared for immunity induction test. Specifically, to HER2/neu E75 peptide (TFA salt) and Montanide ISA51VG (Freund Corporation) as an adjuvant in an amount as shown in Table 5 were added 0.5 parts by weight of the additive (DMSO) and a physiological saline solution as a base material so that the total amount is 100 parts by weight and then blended to form an injection formulation. HER2/neu E75 peptide and the cellular immunity induction promoter were purchased from the same supplier as described for liquid formulations for external use and tape preparations.

Immunity Induction Test 4 in Mouse (Injectable Formulation)

The injectable formulations prepared as described above were used to perform an immunity induction test in mouse according to the same method as Immunity Induction Test 1. The dosage was 200 μL, and the administration was performed once, and the spleen was excised 6 days after the administration. The results of the immunity induction test are shown in Table 5. The mouse used was a genetically modified mouse in which the cellular immunity induction by HLA-A* 0201 type MHC-restricted peptide can be evaluated.

Table 5

| | Composition | | | Results of immunization (ELISPOT average spot number) | % Specific Lysis (In vivo CTL assay) |
|---|---|---|---|---|---|
| | base | antigen peptide | cellular immunity induction promoter | | |
| Comparative example 5 | saline | HER2/neu E75 (0.125) | Montanide ISA51VG (50) | 110 | 28 |

In vivo CTL assay

Seven days after final immunization, the spleen cells (target cell or control cell) were transplanted according to the following procedure, and then, the spleen was isolated after 18 hours. The % Specific Lysis was obtained by performing the FACS measurement.

Procedure 1. Collecting spleen cells of naive mouse

Naive mouse that is the same kind mouse as that used in the immunization test was used. Spleen was isolated from the naive mouse and mashed using a glass slide in a petri dish containing RPMI1640 medium. The mashed spleen was put into a 50 mL tube and centrifuged at 10°C and 1100 rpm for 5 minutes. The supernatant was discarded. 20 mL of Lysis Buffer was added to the tube, followed by incubation at room temperature for 5 minutes. 20 mL of the medium was added to the tube and the tube was then centrifuged. The medium was added to the tube and the resultant was passed through a cell strainer to give spleen cell suspension.

Procedure 2. Labeling spleen cells with an antigen

The spleen cells prepared in Procedure 1 were centrifuged at 10°C and 1100 rpm for 5 minutes, the supernatant was discarded, and HBSS buffer was added to give cell suspension of $2 \times 10^7$ cells/mL. The cell suspension was dispensed into two 50 mL tubes, 100 μM of the antigen solution (the antigen was the same antigen used in the immunization test) was added to one of the tubes containing the cell solution so that the final concentration became 10 μM, to obtain a target cell. The cell in another tube was adopted as control. The cells in both tubes were incubated at 37°C for 1 hour, centrifuged, the supernatant was discarded, and a medium was added.

Procedure 3. Labeling spleen cells with CFSE

The cell labelled with the antigen according to Procedure 2 was centrifuged, and 0.1% BSA-PBS was added to $1 \times 10^7$ cells/mL. To the target cell suspension was added 5 mM CFSE solution to give the final concentration of 10 μM, and to the control cell suspension was added 5 mM CFSE solution to give the final concentration of 1 μM, and the mixture was vortexed, followed by incubation at 37°C for 10 minutes. Thereafter, centrifugation was performed, the supernatant was discarded, and the medium was added.

Procedure 4. Transplanting spleen cells

The cell labelled with CFSE according to Procedure 3 was centrifuged, the supernatant was discarded, and HBSS buffer was added to the cells to give cell suspension of 5 × $10^7$ cells/mL. Equal amounts of the target cell suspension and the control cell suspension were mixed, and 200 μL aliquot of the mixture was introduced into each immunized mouse via orbital veins (transplanted cell number: 1 × $10^7$ cells/animal).

Procedure 5. Preparation of spleen cells of immunized mouse and FACS measurement

Eighteen hours after the transplantation of the spleen cells, spleen of the mouse was isolated, and spleen cell suspension was prepared in the same manner as in Procedure 1. Thereafter, CFSE-positive cells were detected by FACS, and the ratio between CFSE high cells (target cells) and CFSE low cells (control cells) was obtained. The cytotoxic activity was calculated by the formula shown below. The obtained value can be used as an index showing the ability of the antigen specific killer cells induced by the immunization with the vaccine composition to attack specifically the cells that present the antigen in the living body. It was confirmed that the composition of the present invention can induce strong antigen-specific cellular immunity.

$$r = (\%\ \text{CFSE low cells})/(\%\ \text{CFSE high cells})$$

$$\%\ \text{Specific Lysis} = (1 - (r_{\text{non immunized}}/r_{\text{immunized}})) \times 100$$

In Tables 1, 2 and 4, cellular immunity induction promoters were evaluated in regard to a cancer vaccine composition for transdermal administration comprising HER2/neu E75 peptide and/or a modified HER2/neu E75 peptide.

It was found that a first cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more of them was effective.

Preferably, a cellular immunity induction promoter selected from TLR ligand, cyclic dinucleotide, immunomodulatory small molecule drug, cyclooxygenase inhibitor, prostaglandin receptor antagonist, prostaglandin receptor agonist, TSLPproduction inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more of them, as well as a combination of helper peptide with the first cellular immunity induction promoter other than helper peptide were effective. More preferably, a first cellular immunity induction promoter selected from TLR ligand, cyclic dinucleotide, immunomodulatory small molecule drug, cyclooxygenase inhibitor, TSLP production inhibitor, prostaglandin receptor antagonist, prostaglandin receptor agonist, PPAR agonist, TGF-beta production inhibitor, histamine receptor agonist, histamine receptor antagonist, serotonin receptor agonist, serotonin receptor antagonist, muscarine receptor antagonist, adrenalin receptor antagonist, opioid receptor agonist, melatonin receptor agonist, metabotropic glutamate receptor agonist and a combination of two or more of them, as well as a combination of helper peptide with the first cellular immunity induction promoter other than helper peptide were effective.

Further, it was found that the induction of cellular immunity is promoted by the addition of a pharmacologically acceptable acid or a pharmacologically acceptable salt thereof as a second cellular immunity induction promoter.

Specifically, isostearic acid, decanoic acid, lauric acid, palmitic acid, stearic acid, myristic acid, oleic acid, lactic acid, malic acid, maleic acid, salicylic acid and methanesulfonic acid were effective.

Preferably, isostearic acid, decanoic acid, lauric acid, palmitic acid, stearic acid, myristic acid, oleic acid, lactic acid, salicylic acid and methanesulfonic acid were effective.

## Claims

1. A cancer vaccine composition for transdermal administration for use in the induction of cellular immunity, comprising:

   (i) HER2/neu E75 peptide and/or a modified HER2/neu E75 peptide; and
   (ii) a first cellular immunity induction promoter selected from the group consisting of a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, and a combination of two or more them.

2. The cancer vaccine composition for transdermal administration according to claim 1, wherein the composition is for the treatment of a cancer.

3. The cancer vaccine composition for transdermal administration according to claim 1 or 2, further comprising a second cellular immunity induction promoter that is a pharmacologically acceptable acid or a pharmacologically acceptable salt thereof.

4. The cancer vaccine composition for transdermal administration according to any one of claims 1 to 3, wherein the first cellular immunity induction promoter is a helper peptide.

5. The cancer vaccine composition for transdermal administration according to any one of claims 1 to 3, wherein the

first cellular immunity induction promoter is a combination of a helper peptide and at least one substance selected from the group consisting of a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor and a Th2 cytokine inhibitor.

**6.** The cancer vaccine composition for transdermal administration according to any one of claims 1 to 5, wherein the composition is administered under a mildly irritating condition.

**7.** The cancer vaccine composition for transdermal administration according to claim 6, wherein the mildly irritating condition is a condition under which transepidermal water loss (TEWL) in a model animal for skin irritation evaluation before the administration of the composition is 50 $g/h \cdot m^2$ or less.

**8.** The cancer vaccine composition for transdermal administration according to claim 6 or 7, wherein the mildly irritating condition is a condition under which the cutaneous TSLP level in a model animal for skin irritation evaluation at completion of the administration of the composition is 10000 pg/mg protein or less.

**9.** A tape preparation, comprising: a support and an adhesive layer disposed on one side of the support, wherein the adhesive layer comprises the cancer vaccine composition according to any one of claims 1 to 8.

**10.** The cancer vaccine composition for transdermal administration according to any one of claims 1 to 8, wherein the composition is formulated as a liquid formulation for external use.

**11.** The cancer vaccine composition for transdermal administration according to any one of claims 1 to 8, wherein the composition is formulated as a cream formulation.

Europäisches Patentamt
European Patent Office
Office européen des brevets

# EUROPEAN SEARCH REPORT

Application Number
EP 14 00 0323

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOSOI AKIHIRO ET AL: "Memory Th1 cells augment tumor-specific CTL following transcutaneous peptide immunization", CANCER RESEARCH, vol. 68, no. 10, May 2008 (2008-05), pages 3941-3949, XP055109896, ISSN: 0008-5472<br>* page 3941 - page 3942, paragraph top *<br>* page 3944; figure 3 *<br>----- | 1-11 | INV.<br>A61K39/00 |
| A | WO 2008/150577 A1 (JACKSON H M FOUND MILITARY MED [US]; PEOPLES GEORGE E [US]; SATHIBALAN) 11 December 2008 (2008-12-11)<br>* paragraph [0063] *<br>* paragraph [0067] *<br>* paragraph [0087] *<br>* paragraph [0126] *<br>----- | 1-11 | |

TECHNICAL FIELDS SEARCHED (IPC)
A61K

The present search report has been drawn up for all claims

1

| Place of search | Date of completion of the search | Examiner |
|---|---|---|
| The Hague | 28 March 2014 | Mata Vicente, Teresa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 00 0323

This annex lists the patent family members relating to the patent documents cited in the above-mentioned European search report. The members are as contained in the European Patent Office EDP file on
The European Patent Office is in no way liable for these particulars which are merely given for the purpose of information.

28-03-2014

| Patent document cited in search report | Publication date | Patent family member(s) | Publication date |
|---|---|---|---|
| WO 2008150577 A1 | 11-12-2008 | AU 2008260399 A1 | 11-12-2008 |
| | | CA 2687368 A1 | 11-12-2008 |
| | | CN 101678093 A | 24-03-2010 |
| | | DK 2162149 T3 | 10-02-2014 |
| | | EP 2162149 A1 | 17-03-2010 |
| | | EP 2722336 A1 | 23-04-2014 |
| | | ES 2445399 T3 | 03-03-2014 |
| | | HR P20140102 T1 | 28-03-2014 |
| | | JP 2010529026 A | 26-08-2010 |
| | | KR 20100027187 A | 10-03-2010 |
| | | PT 2162149 E | 12-02-2014 |
| | | US 2010209443 A1 | 19-08-2010 |
| | | US 2013189290 A1 | 25-07-2013 |
| | | WO 2008150577 A1 | 11-12-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080193487 A
- JP 2002531415 A
- US 20080112974 A
- JP 7505883 A
- JP 2007529531 A
- WO 9850399 A
- US 6303347 B
- US 6764840 B


### Non-patent literature cited in the description


- **HOSOI AKIHIRO et al.** *Cancer Research,* 2008, vol. 68, 3941-3949
- **ZHENGRONG CUI et al.** *Pharmaceutical Research,* 2002, vol. 19 (7), 947-953
- **MITTENDORF EA et al.** *Cancer Immunol Immunother,* 2008, vol. 57 (10), 1511-1521
- **GEORGE EP et al.** *Journal of Clinical Oncology,* 2005, vol. 23 (30), 7536-7545
- **KAWAI et al.** *Nucleic Acids Research,* vol. 3, 103-4
- Japanese Pharmacopoeia

SEQUENCE LISTING

<110>  NITTO DENKO CORPORATION

<120>  VACCINE COMPOSITION FOR TRANSDERMAL ADMINISTRATION

<130>  NITTO 3

<150>  JP 2013-020799
<151>  2013-02-05

<160>  4

<170>  PatentIn version 3.2

<210>  1
<211>  9
<212>  PRT
<213>  Homo sapiens

<400>  1

```
Lys Ile Phe Gly Ser Leu Ala Phe Leu
1               5
```

<210>  2
<211>  20
<212>  DNA
<213>  Unknown

<220>
<223>  bacterial DNA

<400>  2

```
tccatgacgt tcctgacgtt                                                   20
```

<210>  3
<211>  15
<212>  PRT
<213>  Mycobacterium tuberculosis

<400>  3

```
Phe Gln Asp Ala Tyr Asn Ala Ala Gly Gly His Asn Ala Val Phe
1               5                   10                  15
```

<210>  4
<211>  13
<212>  PRT

<213> Artificial sequence

<220>
<223> Synthetic peptide

<220>
<221> MOD_RES
<222> (1)..(1)
<223> D-alanine

<220>
<221> MOD_RES
<222> (3)..(3)
<223> cyclohexylalanine

<220>
<221> MOD_RES
<222> (13)..(13)
<223> D-alanine

<400> 4

```
Ala Lys Ala Val Ala Ala Trp Thr Leu Lys Ala Ala Ala
1               5                   10
```